# EUROPEAN PATENT APPLICATION

(11) **EP 4 567 087 A1**
(43) Date of publication of application: **11.06.2025**
(21) Application number: 23856552.7
(22) Date of filing: 18.08.2023
(51) Int. Cl.: C09K 11/02, C09K 11/88, H10K 85/30

(54) **NANOCRYSTALLINE COMPOSITE, PREPARATION METHOD THEREFOR, AND USE THEREOF**

(30) Priority: 22.08.2022 CN 202211008516
(71) Applicant: Huawei Technologies Co., Ltd., Shenzhen, Guangdong 518129 (CN); Najing Technology Corporation Limited, Hangzhou, Zhejiang 310052 (CN)
(72) Inventor: LI, Zhe, Shenzhen, Guangdong 518129 (CN); GAO, Yuan, Hangzhou, Zhejiang 310052 (CN); DING, Xianyu, Hangzhou, Zhejiang 310052 (CN)
(74) Representative: Thun, Clemens
(86) International application number: PCT/CN2023/113820
(87) International publication number: WO 2024/041462

(57) **Abstract**

Provided is a nanocrystalline complex, including nanocrystalline particles and a ligand coordinated to a surface of the nanocrystalline particles, and the ligand includes a photosensitive ligand with a structure shown in formula (I) and/or formula (II). X is a coordinating group, Y and Y' are linking groups, one of A and B is -C=O-, the other is a group containing a carbon-carbon double bond, and R, R', and R" are independently a hydrogen atom, a halogen atom, a nitro group, a hydroxyl group, a substituted or unsubstituted alkyl group, a substituted or unsubstituted alkoxy group, a substituted or unsubstituted alkyl carbonyl group, a substituted or unsubstituted cycloalkyl group, a substituted or unsubstituted aryl group, or a substituted or unsubstituted aryloxy group; and n is 1, 2, or 3. The nanocrystalline complex has high photo-crosslinking activity and thermal stability, and is used for preparing a patterned film.

## Description

This application claims priority to Chinese Patent Application No. 202211008516.1, filed with the China National Intellectual Property Administration on August 22, 2022 and entitled "NANOCRYSTALLINE COMPLEX, PREPARATION METHOD THEREFOR, AND USE THEREOF", which is incorporated herein by reference in its entirety.

### TECHNICAL FIELD

Embodiments of this application relate to the field of nanocrystalline film preparation technologies, and in particular, to a nanocrystalline complex, a preparation method therefor, and use thereof.

### BACKGROUND

In the future, AR (Augmented Reality) glasses and VR (Virtual Reality) glasses require a micro-display optical engine that is with ultra-high resolution (≥ 3000 ppi) and that needs to meet all requirements such as ultra-high resolution, low power consumption, highly collimated light emitting, and a small size. Quantum dots are a new type of display materials, characterized by high luminous efficiency and narrow peak width, which can achieve low power consumption, highly collimated light emitting, and a small size.

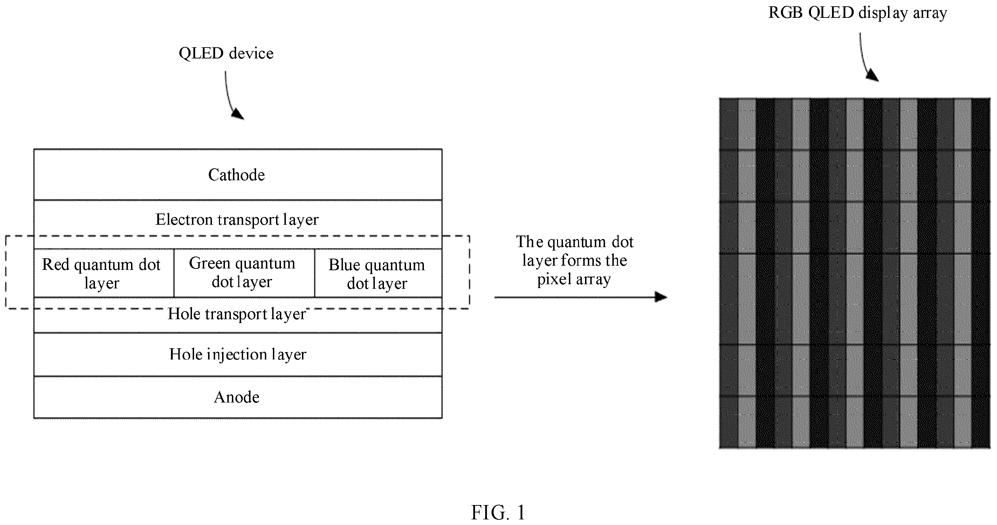

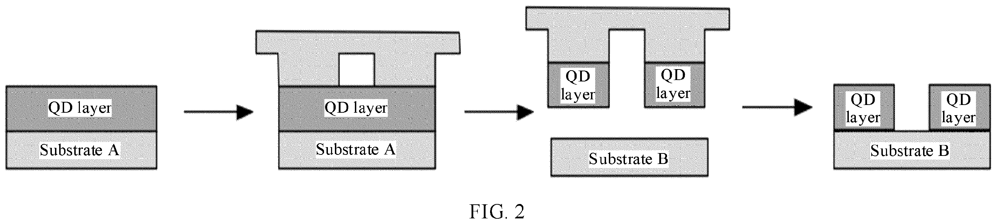

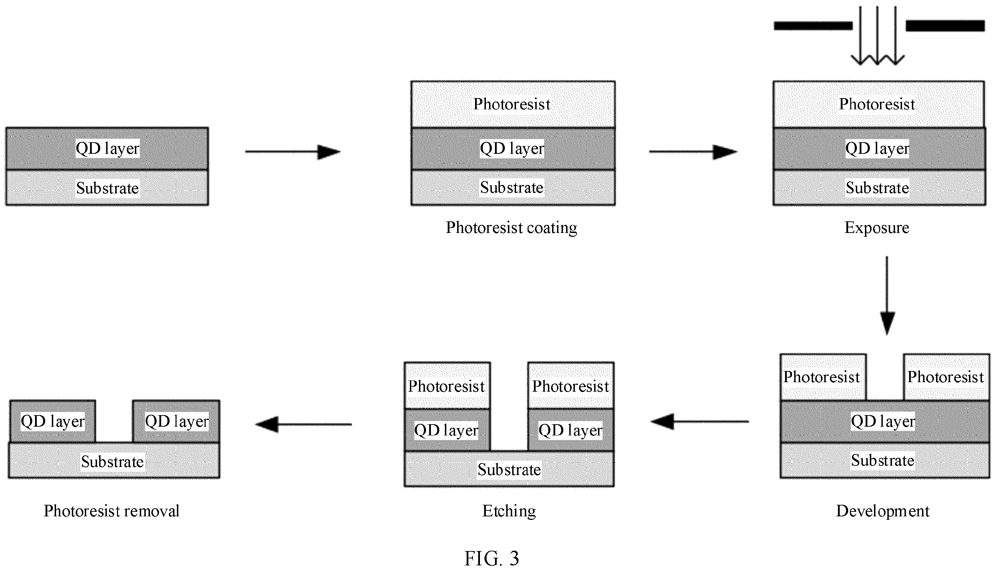

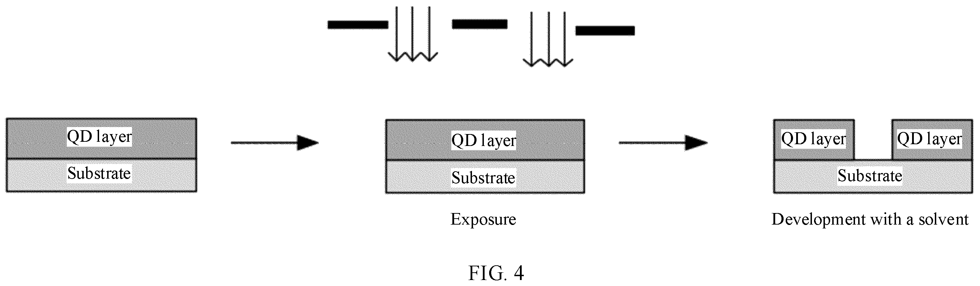

However, to manufacture a display apparatus with ultra-high resolution by using a quantum dot material, fine patterning needs to be performed on a quantum dot (QD) layer of a quantum dot light-emitting device (QLED) to obtain a pixel array (as shown in FIG. 1). Currently, methods for performing fine patterning on the quantum dot layer in the industry are mainly as follows: (1) Transfer printing method: Refer to FIG. 2. The transfer printing method is sticking a quantum dot layer formed on a substrate A away according to a target pattern by using a patterned template, and transferring the quantum dot layer to a substrate B to obtain a patterned quantum dot layer. This method is limited in a processing size, and has a great challenge in a processing yield. (2) Photoresist etching method: Refer to FIG. 3. The photoresist etching method is coating a quantum dot layer with a layer of photoresist, and then performing exposure, development, and etching to obtain a patterned quantum dot layer. This method has many process steps, and a developing solution and an etching solution affect the quantum dot layer and a functional layer (such as a hole transport layer, a hole injection layer, or an electron transport layer). To obtain a high-quality patterned quantum dot layer more conveniently, the industry has begun to attempt to directly synthesize a quantum dot material with a patterning function. Refer to FIG. 4. By using the quantum dot material with the patterning function, a quantum dot layer may be directly subjected to two steps: exposure and development with a solvent to obtain a patterned quantum dot layer. However, some of existing developed quantum dot materials with a patterning function have low photoactivity, requiring long exposure time and a high exposure dose. Some of them have excessively high thermal crosslinking activity, and therefore crosslinking also occurs in the case of heating. In this case, a quantum dot layer is prone to partial crosslinking in an annealing step before exposure, and consequently the quantum dot layer in a non-exposure region cannot be completely developed and eluted, resulting in a residue of the quantum dot layer in the non-exposure region.

### SUMMARY

In view of this, embodiments of this application provide a nanocrystalline complex. The nanocrystalline complex has high photo-crosslinking activity and high thermal stability (that is, low thermal crosslinking activity). The nanocrystalline complex may be used for preparing a patterned film.

Specifically, according to a first aspect of embodiments of this application, a nanocrystalline complex is provided. The nanocrystalline complex includes nanocrystalline particles and a ligand coordinated to a surface of the nanocrystalline particles, and the ligand includes a photosensitive ligand with a structure shown in formula (I) and/or formula (II).

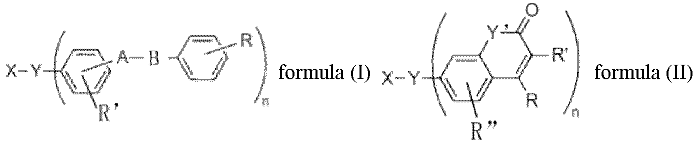

In formula (I), X is a coordinating group coordinated to the nanocrystalline particles, Y is a linking group, one of A and B is -C=O-, the other is a group containing a carbon-carbon double bond, and R and R' are independently one of a hydrogen atom, a halogen atom, a nitro group, a hydroxyl group, a substituted or unsubstituted alkyl group, a substituted or unsubstituted alkoxy group, a substituted or unsubstituted alkyl carbonyl group, a substituted or unsubstituted cycloalkyl group, a substituted or unsubstituted aryl group, and a substituted or unsubstituted aryloxy group;
in formula (II), X is a coordinating group coordinated to the nanocrystalline particles, Y and Y' are linking groups, and R, R', and R" are independently one of a hydrogen atom, a halogen atom, a nitro group, a hydroxyl group, a substituted or unsubstituted alkyl group, a substituted or unsubstituted alkoxy group, a substituted or unsubstituted alkyl carbonyl group, a substituted or unsubstituted cycloalkyl group, a substituted or unsubstituted aryl group, and a substituted or unsubstituted aryloxy group; and
n is 1, 2, or 3.

In embodiments of this application, the nanocrystalline complex with high photosensitivity and high thermal stability is obtained by coordinating the photosensitive ligand of the structure shown in formula (I) and/or formula (II) with high photo-crosslinking activity and low thermal crosslinking activity to the surface of the nanocrystalline particles. The nanocrystalline complex has a patterning function. The nanocrystalline complex is used for preparing a patterned nanocrystalline film, so that patterning can be implemented directly through exposure and development with a solvent. In a patterning process, the nanocrystalline complex can undergo photo-crosslinking with a small exposure dose and short exposure time. Specifically, crosslinking between the nanocrystalline complexes is implemented by using the photosensitive ligand, and no additional photoinitiator is required in the process of implementing photo-crosslinking. In addition, thermal crosslinking does not occur when the nanocrystalline complex undergoes heating and annealing processes, so that a problem of incomplete elution in a non-exposure region in a development process can be avoided, improving reliability of a patterning process, and facilitating fine preparation of a pattern.

In some implementations of this application, the ligand 102 on the surface of the nanocrystalline particles 101 may include one or more photosensitive ligands with the structure shown in formula (I). In some implementations of this application, the ligand 102 on the surface of the nanocrystalline particles 101 may include one or more photosensitive ligands with the structure shown in formula (II). In some implementations of this application, the ligand 102 on the surface of the nanocrystalline particles 101 may include one or more photosensitive ligands with the structure shown in formula (I), and/or one or more photosensitive ligands with the structure shown in formula (II). In some implementations of this application, the ligand 102 on the surface of the nanocrystalline particles 101 may further include a ligand with another structure or another function.

In an implementation of this application, the coordinating group includes any one of a carboxyl group, an amino group, a phosphate group, a phospholipid group, and a mercapto group.

In an implementation of this application, the linking group Y includes one or more of a substituted or unsubstituted alkylene group, a substituted or unsubstituted arylene group, a substituted or unsubstituted arylene alkyl group, a substituted or unsubstituted alkylene aryl group, a substituted or unsubstituted alkyleneoxy group, a substituted or unsubstituted aryleneoxy group, a substituted or unsubstituted alkylene aryloxy group, a substituted or unsubstituted arylene alkoxy group, a carbonyl-containing group, an ester-containing and/or ether-oxygen bond-containing group, and an imino-containing group.

The carbonyl-containing group includes any one of -C(=O)- and -R₁-C(=O)-; the ester-containing and/or ether-oxygen bond-containing group includes any one of -R₂-C(=O)-O-, 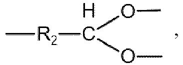 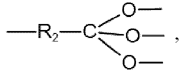 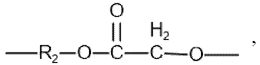 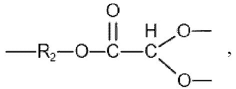 and 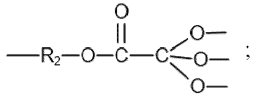 and the imino-containing group may be any one of -R₃-CH₂-NH-, 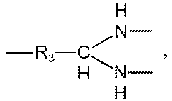 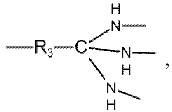 and -R₃-C(=O)-NH-, where R₁, R₂, and R₃ are substituted or unsubstituted alkylene groups.

In an implementation of this application, a number of carbon atoms in the linking group Y is 1 to 30. An appropriate number of carbon atoms in the linking group Y can well balance dispersion of quantum dots and conductivity of a quantum dot layer.

In an implementation of this application, the linking group Y' includes one or more of an oxygen atom, a sulfur atom, a substituted or unsubstituted alkylene group, a substituted or unsubstituted alkyleneoxy group, an ester-containing group, and an amido bond-containing group. The ester-containing group may be -R₁-C(=O)-O-, and the amido bond-containing group may be -R₃-C(=O)-NH-, where R₁ and R₃ are substituted or unsubstituted alkylene groups.

In an implementation of this application, a number of carbon atoms in the linking group Y' is 0 to 30.

In an implementation of this application, in R, R', and R", the substituted or unsubstituted alkyl group is a substituted or unsubstituted C1-C20 alkyl group, the substituted or unsubstituted alkoxy group is a substituted or unsubstituted C1-C20 alkoxy group, the substituted or unsubstituted alkyl carbonyl group is a substituted or unsubstituted C1-C20 alkyl carbonyl group, the substituted or unsubstituted cycloalkyl group is a substituted or unsubstituted C3-C20 cycloalkyl group, the substituted or unsubstituted aryl group is a substituted or unsubstituted C6-C20 aryl group, and the substituted or unsubstituted aryloxy group is a substituted or unsubstituted C6-C20 aryloxy group.

In an implementation of this application, the photosensitive ligand with the structure shown in formula (I) includes any one of compounds shown in formula (I-1) to formula (I-14):

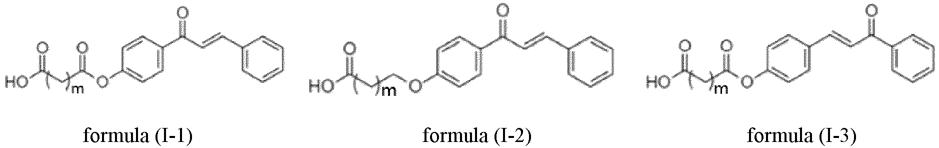

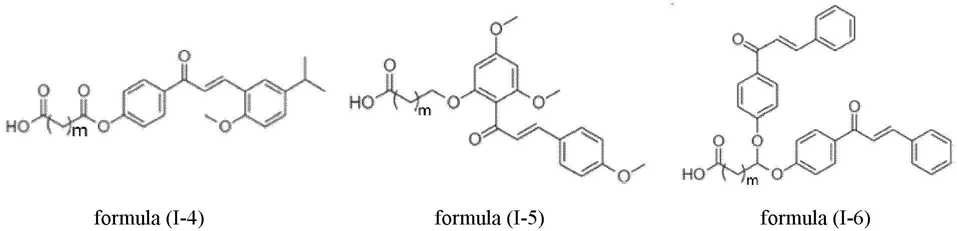

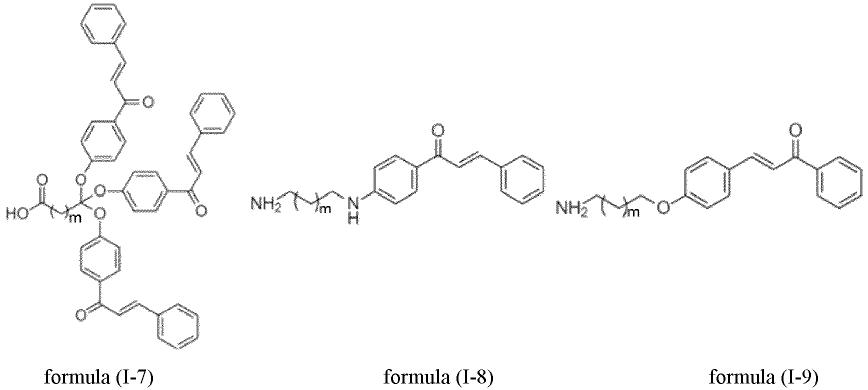

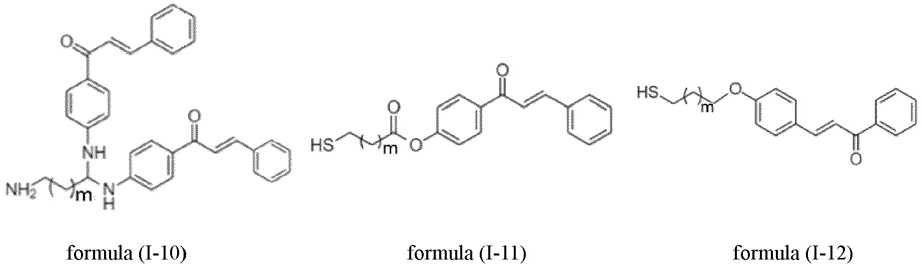

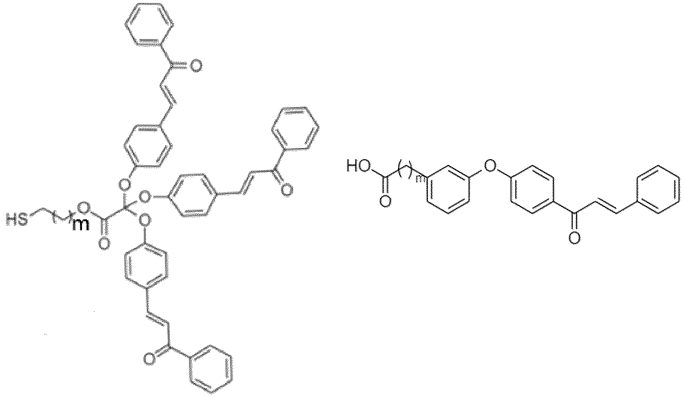

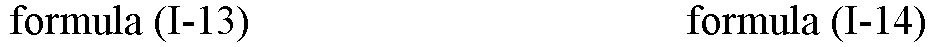

In formula (I-1) to formula (I-14), m is an integer greater than or equal to 0.

In an implementation of this application, the photosensitive ligand with the structure shown in formula (II) includes any one of compounds shown in formula (II-1) to formula (II-12):

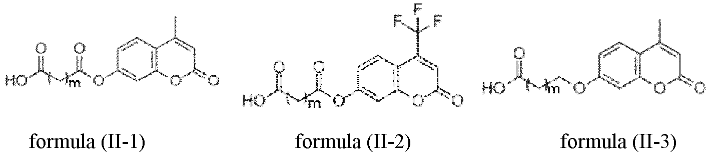

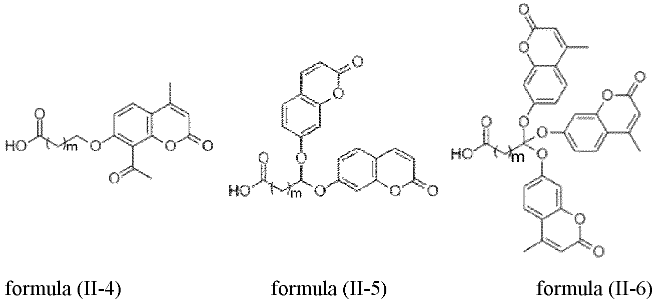

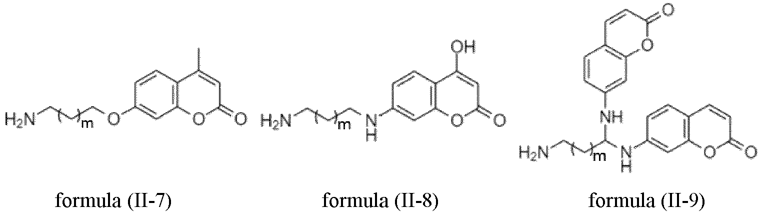

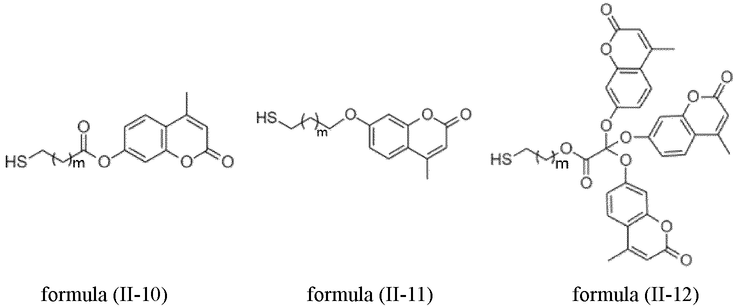

In formula (II-1) to formula (II-12), m is an integer greater than or equal to 0.

In an implementation of this application, the nanocrystalline particles include one or more of a group II-VI compound, a group IV-VI compound, a group III-V compound, a group III-VI compound, a group VIB-VIA compound, a group VIII-VI compound, a group I-VI compound, a group I elementary substance, a group IV elementary substance, a group I-IV-VII compound, and a perovskite compound.

In an implementation of this application, a particle size of the nanocrystalline particles is 5 nm to 20 nm. An appropriate particle size of the nanocrystalline particles is conducive to obtaining good luminescence performance.

In an implementation of this application, a total mass of the ligand on the surface of the nanocrystalline particles is 1% to 30% of a mass of the nanocrystalline particles. An appropriate coordination amount of the photosensitive ligand helps the nanocrystalline complex to have high photosensitivity and high thermal stability, and helps well utilize properties of the nanocrystalline particles.

According to a second aspect of embodiments of this application, a method for preparing the nanocrystalline complex according to the first aspect is provided, including:
dispersing nanocrystalline particles whose surface is coordinated with a first ligand in a solvent, adding the photosensitive ligand with the structure shown in formula (I) and/or formula (II), and stirring to undergo a reaction, to obtain ligand-exchanged nanocrystalline particles, that is, to obtain the nanocrystalline complex.

In an implementation of this application, the first ligand includes a saturated or unsaturated aliphatic carboxylic acid ligand.

In an implementation of this application, the solvent includes one or more of chloroform, chlorobenzene, and ethyl benzoate.

The method for preparing the nanocrystalline complex provided in embodiments of this application has a simple process, which can easily implement quantitative production.

According to a third aspect of embodiments of this application, a nanocrystalline composition is provided. The nanocrystalline composition includes the nanocrystalline complex according to the first aspect of embodiments of this application and a solvent.

In an implementation of this application, in the nanocrystalline composition, a mass concentration of the nanocrystalline complex is 1 mg/mL to 100 mg/mL. An appropriate concentration of the nanocrystalline complex can help a nanocrystalline film prepared by using the nanocrystalline composition to have good comprehensive performance.

In an implementation of this application, the solvent includes one or more of chloroform, chlorobenzene, and ethyl benzoate.

According to a fourth aspect of embodiments of this application, a method for preparing a patterned film is provided, including:
providing a solution containing the nanocrystalline complex according to the first aspect of embodiments of this application or providing the nanocrystalline composition according to the third aspect of embodiments of this application on a substrate, and performing exposure and development by using a mask, where the nanocrystalline complex in an exposure region undergoes photo-crosslinking to be attached to the substrate, and the nanocrystalline complex in a non-exposure region is removed through the development, to form the patterned film.

In an implementation of this application, the exposure is performed through radiation of ultraviolet light with a light intensity of 0.001 mW/cm² to 1000 mW/cm².

In an implementation of this application, time for the exposure is 0.1 seconds to 600 seconds.

In an implementation of this application, the development is development with a solvent, and an organic solvent capable of eluting the nanocrystalline complex is used for the development.

According to the method for preparing the patterned film in embodiments of this application, good crosslinking effect can be achieved with short exposure time, and only development with a solvent is required, which makes a process simple. This can not only save energy and time, but also obtain a pattern with high resolution.

According to a fifth aspect of embodiments of this application, provided is a patterned film formed through crosslinking of the nanocrystalline complex according to the first aspect of embodiments of this application; or a patterned film prepared by the preparation method according to the fourth aspect of embodiments of this application.

According to a sixth aspect of embodiments of this application, an electronic component is provided. The electronic component includes a first electrode and a second electrode that are opposite to each other, and a functional layer between the first electrode and the second electrode. The functional layer includes a crosslinking product of the nanocrystalline complex according to the first aspect of embodiments of this application; or the functional layer includes the patterned film according to the fifth aspect of embodiments of this application.

In an implementation of this application, the electronic component includes any one of an LED, a QLED, a mini-LED, a micro-LED, a nano-LED, and a QD-OLED.

According to a seventh aspect of embodiments of this application, a display apparatus is further provided. The display apparatus includes the electronic component according to the sixth aspect of embodiments of this application.

An embodiment of this application further provides an electronic device. The electronic device includes the display apparatus according to the seventh aspect.

### BRIEF DESCRIPTION OF THE DRAWINGS

FIG. 1 is a diagram of performing fine patterning on a quantum dot layer to obtain a pixel array;
FIG. 2 is a diagram of a process of patterning a quantum dot layer by a transfer printing method;
FIG. 3 is a diagram of a process of patterning a quantum dot layer by a photoresist etching method;
FIG. 4 is a diagram of a process of patterning a quantum dot layer formed from a quantum dot material with a patterning function;
FIG. 5 is a diagram of a structure of a nanocrystalline complex 100 according to an embodiment of this application;
FIG. 6 is a diagram of photo-crosslinking between nanocrystalline complex particles according to an embodiment of this application;
FIG. 7 is a schematic flowchart of a method for preparing a patterned film according to an embodiment of this application;
FIG. 8 is a diagram of a structure of an electronic component 200 according to an embodiment of this application;
FIG. 9 is a diagram of a structure of a display apparatus 300 according to an embodiment of this application;
FIG. 10 is a diagram of a structure of an electronic device 400 according to an embodiment of this application;
FIG. 11 is a nuclear magnetic resonance spectrum of a photosensitive ligand ChalBen prepared according to Embodiment 1 of this application;
FIG. 12 is a nuclear magnetic resonance spectrum of a photosensitive ligand ChalC5 prepared according to Embodiment 2 of this application;
FIG. 13 is a nuclear magnetic resonance spectrum of a photosensitive ligand ChalC8 prepared according to Embodiment 3 of this application;
FIG. 14 is a nuclear magnetic resonance spectrum of a photosensitive ligand ChalC13 prepared according to Embodiment 4 of this application;
FIG. 15 is a nuclear magnetic resonance spectrum of a photosensitive ligand CouC2 prepared according to Embodiment 5 of this application;
FIG. 16 is a nuclear magnetic resonance spectrum of a photosensitive ligand CouC8 prepared according to Embodiment 6 of this application;
FIG. 17 is a nuclear magnetic resonance spectrum of a photosensitive ligand CouC13 prepared according to Embodiment 7 of this application;
FIG. 18 and FIG. 19 show UV-VIS (ultraviolet-visible) absorption curves of a quantum dot film sample at different stages according to an embodiment of this application; and
FIG. 20 and FIG. 21 show UV-VIS (ultraviolet-visible) absorption curves of a quantum dot film sample at different stages according to a comparative example.

### DESCRIPTION OF EMBODIMENTS

The following describes embodiments of this application with reference to accompanying drawings in embodiments of this application.

To manufacture a display apparatus with ultra-high resolution by using a quantum dot material, fine patterning needs to be performed on a quantum dot (QD) layer of a quantum dot light-emitting device (QLED) to obtain a pixel array. Currently, methods for performing fine patterning on the quantum dot layer in the industry mainly include a transfer printing method and a photoresist etching method. However, the transfer printing method is limited in a processing size and poor in a processing yield; and the photoresist etching method has many process steps, and a developing solution and an etching solution affect the quantum dot layer and a functional layer. To more conveniently obtain a high-quality patterned quantum dot layer and meet requirements for manufacturing a display apparatus with high resolution, embodiments of this application provide a nanocrystalline complex. The nanocrystalline complex has high photo-crosslinking activity and high thermal stability (that is, low thermal crosslinking activity). The nanocrystalline complex is used for preparing a patterned film, which has a simple process and good patterning effect, and can avoid the effect of a patterning process on the quantum dot layer and the functional layer.

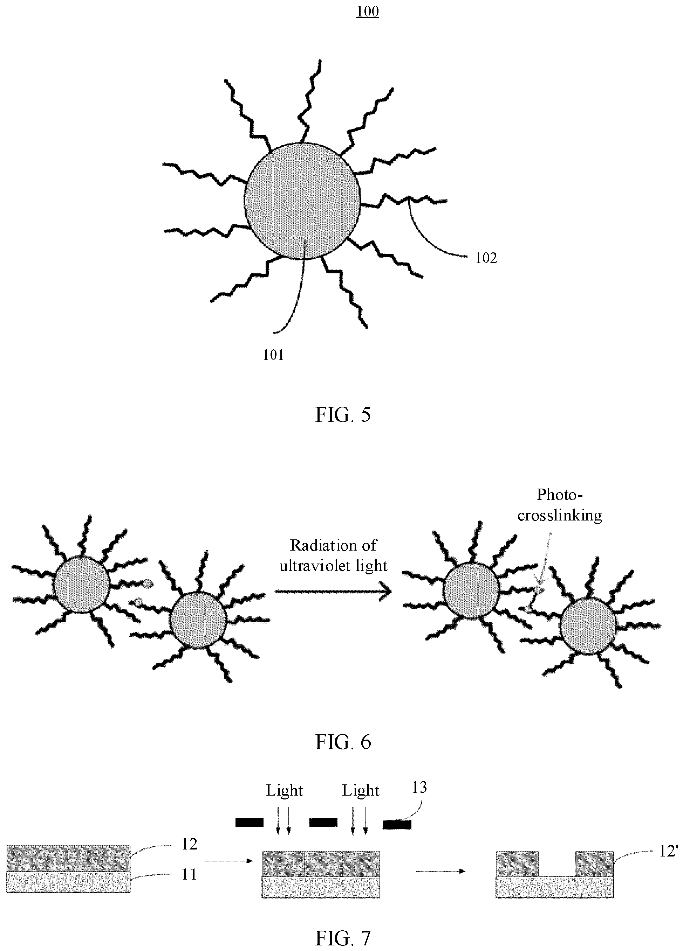

FIG. 5 is a diagram of a structure of a nanocrystalline complex 100 according to an embodiment of this application. The nanocrystalline complex 100 includes nanocrystalline particles 101 and a ligand 102 coordinated to a surface of the nanocrystalline particles 101, and the ligand 102 includes a photosensitive ligand with a structure shown in formula (I) and/or formula (II).

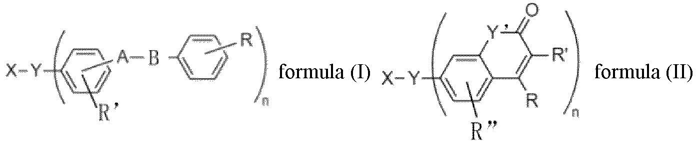

In formula (I), X is a coordinating group coordinated to the nanocrystalline particles 101, Y is a linking group, one of A and B is -C=O-, the other is a group containing a carbon-carbon double bond, and R and R' are independently one of a hydrogen atom, a halogen atom, a nitro group, a hydroxyl group, a substituted or unsubstituted alkyl group, a substituted or unsubstituted alkoxy group, a substituted or unsubstituted alkyl carbonyl group, a substituted or unsubstituted cycloalkyl group, a substituted or unsubstituted aryl group, and a substituted or unsubstituted aryloxy group;
in formula (II), X is a coordinating group coordinated to the nanocrystalline particles 101, Y and Y' are linking groups, and R, R', and R" are independently one of a hydrogen atom, a halogen atom, a nitro group, a hydroxyl group, a substituted or unsubstituted alkyl group, a substituted or unsubstituted alkoxy group, a substituted or unsubstituted alkyl carbonyl group, a substituted or unsubstituted cycloalkyl group, a substituted or unsubstituted aryl group, and a substituted or unsubstituted aryloxy group; and
n may be 1, 2, or 3.

In embodiments of this application, the nanocrystalline complex with high photosensitivity and high thermal stability is obtained by coordinating the photosensitive ligand of the structure shown in formula (I) and/or formula (II) with high photo-crosslinking activity and low thermal crosslinking activity to the surface of the nanocrystalline particles. The nanocrystalline complex has a patterning function. The nanocrystalline complex is used for preparing a patterned nanocrystalline film, so that patterning can be implemented directly through exposure and development with a solvent. In a patterning process, the nanocrystalline complex can undergo photo-crosslinking with a small exposure dose and short exposure time. Specifically, crosslinking between the nanocrystalline complexes is implemented by using the photosensitive ligand, and no additional photoinitiator is required in the process of implementing photo-crosslinking. In addition, thermal crosslinking does not occur when the nanocrystalline complex undergoes heating and annealing processes, so that a problem of incomplete elution in a non-exposure region in a development process can be avoided, improving reliability of a patterning process, and facilitating fine preparation of a pattern.

In some implementations of this application, the ligand 102 on the surface of the nanocrystalline particles 101 may include one or more photosensitive ligands with the structure shown in formula (I). In some implementations of this application, the ligand 102 on the surface of the nanocrystalline particles 101 may include one or more photosensitive ligands with the structure shown in formula (II). In some implementations of this application, the ligand 102 on the surface of the nanocrystalline particles 101 may include one or more photosensitive ligands with the structure shown in formula (I), and/or one or more photosensitive ligands with the structure shown in formula (II). In some implementations of this application, the ligand 102 on the surface of the nanocrystalline particles 101 may further include a ligand with another structure or another function.

In an implementation of this application, in formula (I) and formula (II), X is a coordinating group, that is, a group that forms coordinate bonding with the surface of the nanocrystalline particles 101. The coordinating group X may be various groups that can form coordinate bonding with the surface of the nanocrystalline particles 101, including but not limited to any one of a carboxyl group (-COOH), an amino group (-NH₂), a phosphate group, a phospholipid group, and a mercapto group (-SH). These coordinating groups can help the photosensitive ligand to be well anchored to the surface of inorganic nanocrystalline quantum dots.

In an implementation of this application, in formula (I) and formula (II), the linking group Y includes one or more of a substituted or unsubstituted alkylene group, a substituted or unsubstituted arylene group, a substituted or unsubstituted arylene alkyl group, a substituted or unsubstituted alkylene aryl group, a substituted or unsubstituted alkyleneoxy group, a substituted or unsubstituted aryleneoxy group, a substituted or unsubstituted alkylene aryloxy group, a substituted or unsubstituted arylene alkoxy group, a carbonyl-containing group, an ester-containing and/or ether-oxygen bond-containing group, and an imino-containing group. The carbonyl-containing group includes any one of -C(=O)- and -R₁-C(=O)-; the ester-containing and/or ether-oxygen bond-containing group includes any one of -R₂-C(=O)-O-, 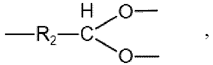 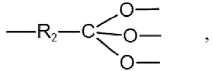 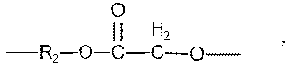 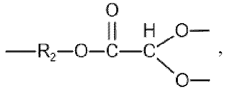 and 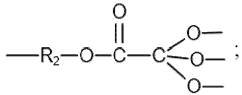 and the imino-containing group may be any one of -R₃-CH₂-NH-, 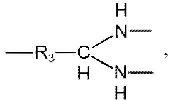 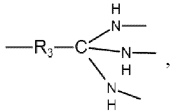 and -R₃-C(=O)-NH-, where R₁, R₂, and R₃ are substituted or unsubstituted alkylene groups. In R₁, R₂, and R₃, a substituent in the substituted alkylene group may be but is not limited to a halogen atom, and may be specifically a fluorine atom, a chlorine atom, a bromine atom, or an iodine atom.

In an implementation of this application, when the linking group Y has a branch structure, n in formula (I) and formula (II) is 2 or 3. Specifically, when the linking group Y is 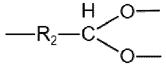 or 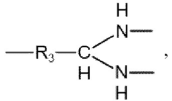 n is 2; and when the linking group Y is 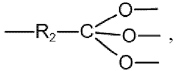 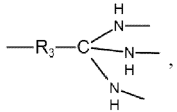 or 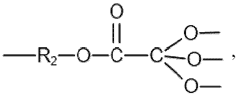 n is 3.

In some implementations of this application, a number of carbon atoms in the linking group Y may be 1 to 30. In some embodiments, the number of carbon atoms in the linking group Y may be specifically 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30. An appropriate number of carbon atoms in the linking group Y can well balance dispersion of quantum dots and conductivity of a quantum dot layer.

In an implementation of this application, in the linking group Y, the substituted or unsubstituted alkylene group may be, for example, a substituted or unsubstituted methylene group (-CH₂-), a substituted or unsubstituted ethylene group, a substituted or unsubstituted propylidene group, a substituted or unsubstituted isopropylidene group, a substituted or unsubstituted butylidene group, a substituted or unsubstituted isobutylidene group, a substituted or unsubstituted neopentylidene group, or a substituted or unsubstituted hexylidene group. The alkylene group is obtained by removing one hydrogen atom from an alkyl group. The substituted or unsubstituted arylene group may be a substituted or unsubstituted arylene group with a number of carbon atoms of 6 to 30, and may be specifically, for example, a substituted or unsubstituted phenylene group (-C₆H₄-), a substituted or unsubstituted biphenylene group, a substituted or unsubstituted triphenylene group, a substituted or unsubstituted fluorenylidene group, a substituted or unsubstituted naphthylene group, or a substituted or unsubstituted anthrylene group. The substituted or unsubstituted alkylene aryl group may have a number of carbon atoms of 7 to 30, and may be specifically an alkylene phenyl group, for example, a methylene phenyl group (-CH₂-C₆H₄-) or an ethylene phenyl group (-CH₂CH₂-C₆H₄-). The substituted or unsubstituted arylene alkyl group may have a number of carbon atoms of 7 to 30, and may be specifically a phenylene alkyl group, for example, a phenylene methyl group (-C₆H₄-CH₂-) or a phenylene ethyl group (-C₆H₄-CH₂CH₂-). The substituted or unsubstituted alkyleneoxy group may be, for example, a substituted or unsubstituted methyleneoxy group (-CH₂-O-), a substituted or unsubstituted ethyleneoxy group (-CH₂CH₂-O-), a substituted or unsubstituted propylideneoxy group (-CH₂CH₂CH₂-O-), a substituted or unsubstituted isopropylideneoxy group, a substituted or unsubstituted butylideneoxy group, a substituted or unsubstituted isobutylideneoxy group, a substituted or unsubstituted neopentylideneoxy group, or a substituted or unsubstituted hexylideneoxy group. The substituted or unsubstituted aryleneoxy group may have a number of carbon atoms of 6 to 30, and may be specifically a phenyleneoxy group (-C₆H₄-O-) or the like. The substituted or unsubstituted alkylene aryloxy group may have a number of carbon atoms of 7 to 30, and may be specifically an alkylene phenoxy group, for example, a methylene phenoxy group (-CH₂-C₆H₄-O-) or an ethylene phenoxy group (-CH₂CH₂-C₆H₄-O-). The substituted or unsubstituted arylene alkoxy group may have a number of carbon atoms of 7 to 30, and may be specifically a phenylene alkyl group, for example, a phenylene methoxy group (-C₆H₄-CH₂-O-) or a phenylene ethoxy group (-C₆H₄-CH₂CH₂-O-).

In an implementation of this application, in the linking group Y, a substituent in the substituted alkylene group, the substituted arylene group, the substituted arylene alkyl group, the substituted alkylene aryl group, the substituted alkyleneoxy group, the substituted aryleneoxy group, the substituted alkylene aryloxy group, the substituted arylene alkoxy group, and the substituted alkylene carbonyl group may be but is not limited to a halogen atom, and may be specifically a fluorine atom, a chlorine atom, a bromine atom, or an iodine atom.

In an implementation of this application, in formula (II), the linking group Y' may include one or more of an oxygen atom, a sulfur atom, a substituted or unsubstituted alkylene group, a substituted or unsubstituted alkyleneoxy group, an ester-containing group, and an amido bond-containing group. The ester-containing group may be -R₁-C(=O)-O-, and the amido bond-containing group may be -R₃-C(=O)-NH-, where R₁ and R₃ are substituted or unsubstituted alkylene groups. In R₁ and R₃, a substituent in the substituted alkylene group may be but is not limited to a halogen atom, and may be specifically a fluorine atom, a chlorine atom, a bromine atom, or an iodine atom.

In some implementations of this application, a number of carbon atoms in the linking group Y' may be 1 to 30. In some embodiments, the number of carbon atoms in the linking group Y' may be specifically 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30. In some embodiments, the linking group Y' is an oxygen atom.

In formula (I), one of A and B is (-C(=O)-), and the other is a group containing a carbon-carbon double bond. The group containing the carbon-carbon double bond may be specifically a substituted or unsubstituted alkenylene group, for example, may be specifically a substituted or unsubstituted ethenylene group (-CH=CH-). In some embodiments, A is (-C(=O)-), and B is a group containing a carbon-carbon double bond. In some embodiments, A is a group containing a carbon-carbon double bond, and B is (-C(=O)-). In some embodiments, A may be linked to a para-position of the linking group Y. In some embodiments, A may be linked to an ortho-position of the linking group Y. In some embodiments, A may be linked to a meta-position of the linking group Y.

In an implementation of this application, the substituents R, R', and R" may be selected from any one of a hydrogen atom, a halogen atom, a nitro group, a hydroxyl group, a substituted or unsubstituted alkyl group, a substituted or unsubstituted alkoxy group, a substituted or unsubstituted alkyl carbonyl group, a substituted or unsubstituted cycloalkyl group, a substituted or unsubstituted aryl group, and a substituted or unsubstituted aryloxy group. When R, R', and R" are a halogen atom, R, R', and R" may be specifically a fluorine atom, a chlorine atom, a bromine atom, or an iodine atom. In an implementation of this application, in R, R', and R", the substituted or unsubstituted alkyl group may be a substituted or unsubstituted C1-C20 alkyl group, and may be specifically a substituted or unsubstituted C1, C2, C3, C4, C5, C6, C7, C8, C9, C10, C11, C12, C13, C14, C15, C16, C17, C18, C19, or C20 alkyl group, such as a substituted or unsubstituted methyl group (-CH₃), a substituted or unsubstituted ethyl group, a substituted or unsubstituted isopropyl group, or a substituted or unsubstituted butyl group; the substituted or unsubstituted alkoxy group may be a substituted or unsubstituted C1-C20 alkoxy group, and may be specifically a substituted or unsubstituted C1, C2, C3, C4, C5, C6, C7, C8, C9, C10, C11, C12, C13, C14, C15, C16, C17, C18, C19, or C20 alkoxy group, such as a substituted or unsubstituted methoxy group (-OCH₃), a substituted or unsubstituted ethoxy group (-OCH₂CH₃), a substituted or unsubstituted propoxy group, or a substituted or unsubstituted butoxy group; the substituted or unsubstituted alkyl carbonyl group may be a substituted or unsubstituted C1-C20 alkyl carbonyl group, and may be specifically a substituted or unsubstituted C1, C2, C3, C4, C5, C6, C7, C8, C9, C10, C11, C12, C13, C14, C15, C16, C17, C18, C19, or C20 alkyl carbonyl group, such as a substituted or unsubstituted methyl carbonyl group (CH₃-(C=O)-), a substituted or unsubstituted ethyl carbonyl group (CH₃CH₂-(C=O)-), a substituted or unsubstituted propyl carbonyl group, or a substituted or unsubstituted butyl carbonyl group; the substituted or unsubstituted cycloalkyl group may be a substituted or unsubstituted C3-C20 cycloalkyl group, such as a substituted or unsubstituted cyclopropyl group, a substituted or unsubstituted cyclobutyl group, a substituted or unsubstituted cyclopentyl group, or a substituted or unsubstituted cyclohexyl group; the substituted or unsubstituted aryl group may be a substituted or unsubstituted C6-C20 aryl group, and may be specifically a substituted or unsubstituted C6, C7, C8, C9, C10, C11, C12, C13, C14, C15, C16, C17, C18, C19, or C20 aryl group, such as a substituted or unsubstituted phenyl, naphthyl, biphenyl, triphenyl, fluorenyl, or anthryl group; and the substituted or unsubstituted aryloxy group may be a substituted or unsubstituted C6-C20 aryloxy group, and may be specifically a substituted or unsubstituted C6, C7, C8, C9, C10, C11, C12, C13, C14, C15, C16, C17, C18, C19, or C20 aryloxy group, such as a substituted or unsubstituted phenoxy group. In R, R', and R", a substituent in the substituted alkyl group, the substituted alkoxy group, the substituted alkyl carbonyl group, the substituted cycloalkyl group, the substituted aryl group, and the substituted aryloxy group may be but is not limited to a halogen atom (including a fluorine atom, a chlorine atom, a bromine atom, or an iodine atom). For example, the substituted alkyl group may be a trifluoromethyl group, a trifluoroethyl group, or the like. In formula (I), R and R' may be on any vacant carbon atom (a carbon atom not linked to the linking group Y, the group A, and the group B) on a ring structure on which R and R' are located. In formula (II), R" may be on any vacant carbon atom on a ring structure on which R" is located.

In some implementations of this application, the photosensitive ligand with the structure shown in formula (I) may specifically include any one of compounds shown in formula (I-1) to formula (I-14):

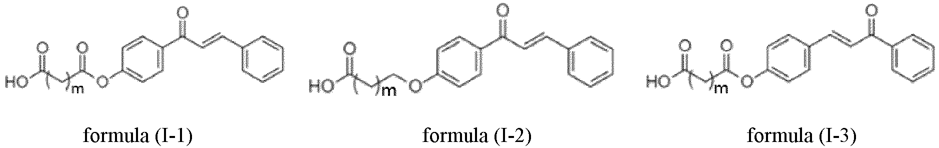

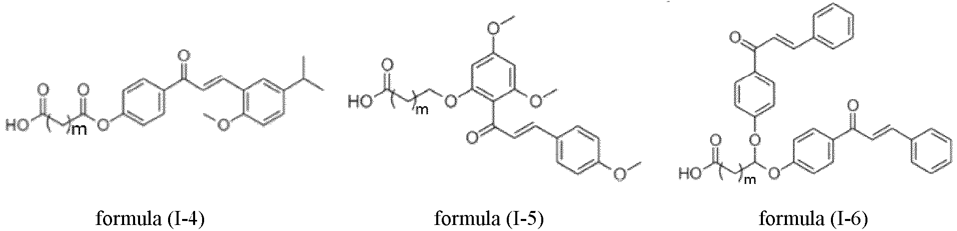

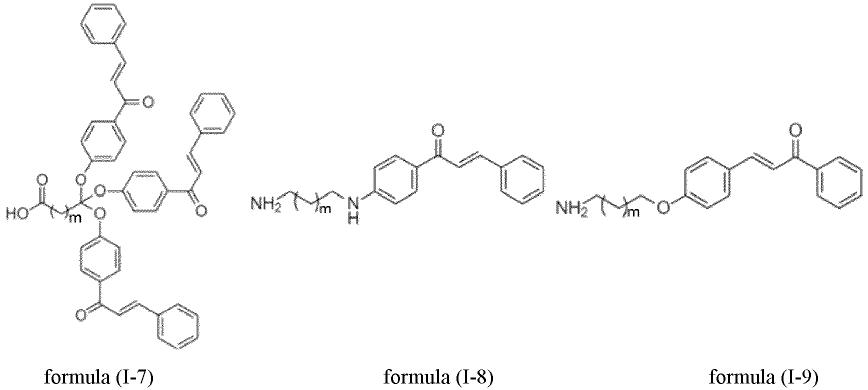

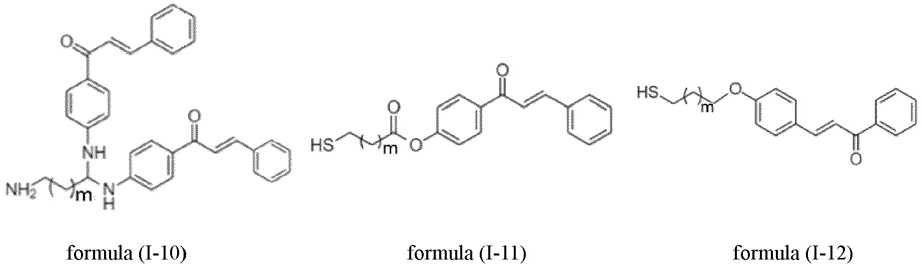

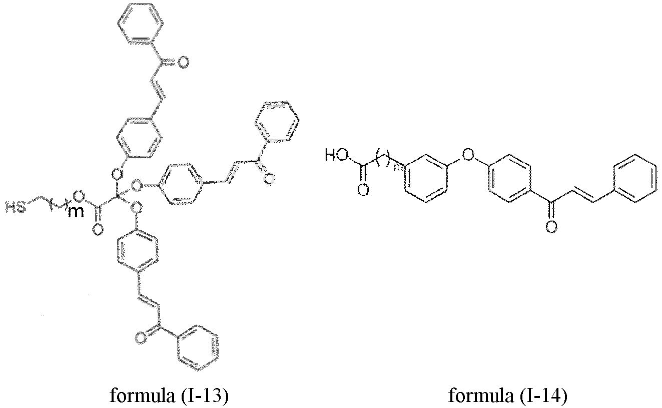

In formula (I-1) to formula (I-14), m is an integer greater than or equal to 0. Specifically, m may be, for example, 0, 1, 2, 3, 4, 5, 6, 7, 8, 9, 10, 11, 12, 13, 14, 15, 16, 17, 18, 19, 20, 21, 22, 23, 24, 25, 26, 27, 28, or 29.

In some implementations of this application, the photosensitive ligand with the structure shown in formula (II) may specifically include any one of compounds shown in formula (II-1) to formula (II-12):

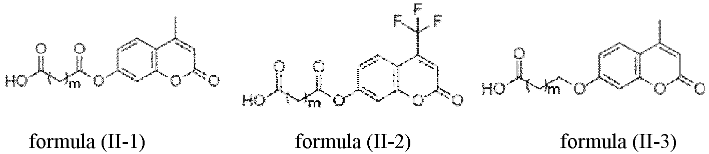

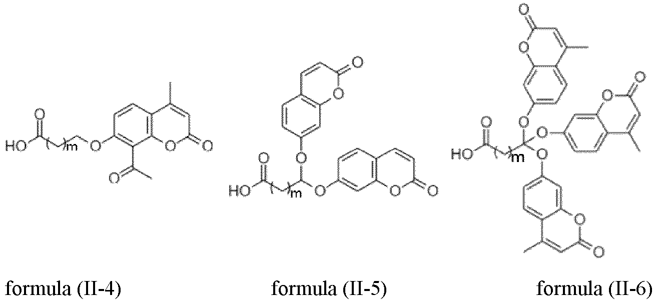

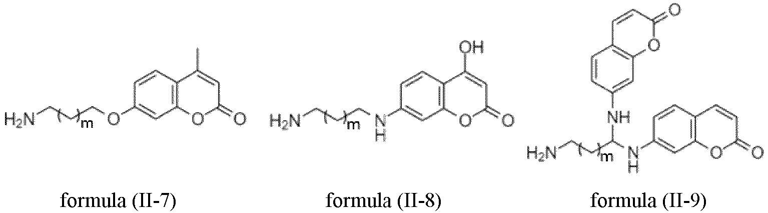

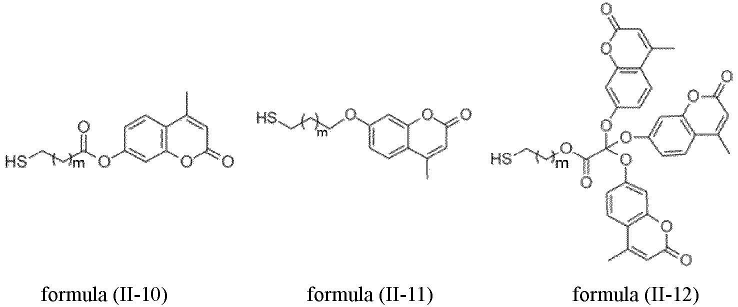

In formula (II-1) to formula (II-12), m is an integer greater than or equal to 0. Specifically, m may be, for example, 0, 1, 2, 3, 4, 5, 6, 7, 8, 9, 10, 11, 12, 13, 14, 15, 16, 17, 18, 19, 20, 21, 22, 23, 24, 25, 26, 27, 28, or 29.

In an implementation of this application, the nanocrystalline particles include one or more of a group II-VI compound, a group IV-VI compound, a group III-V compound, a group III-VI compound, a group VIB-VIA compound, a group VIII-VI compound, a group I-VI compound, a group I elementary substance, a group IV elementary substance, a group I-IV-VII compound, and a perovskite compound. The nanocrystalline particles in embodiments of this application may be composed of one of the foregoing materials, or may be composed of two or more of the foregoing materials. The nanocrystalline particles may be particles of a single material, particles of a uniform mixture, particles of a gradient mixture, particles of a core-shell structure, or the like, or may be a product in which the foregoing compounds are doped.

In some implementations of this application, the nanocrystalline particles may include but are not limited to a CdS, CdSe, CdTe, ZnS, ZnSe, ZnTe, PbS, PbSe, PbTe, HgS, HgSe, HgTe, GaN, GaP, GaAs, InP, InAs, ZnO, SnO₂, TiO₂, In₂O₃, Ga₂O₃, SiO₂, NiO, MoO₃, WO₃, Cu₂O, CuO, Fe₃O₄, Au, Ag, carbon dots, CsPbCl₃, CsPbBr₃, CsPbI₃, CH₃NH₃PbCl₃, CH₃NH₃PbBr₃, CH₃NH₃PbI₃, a mixture, gradient mixture, or core-shell structure of the foregoing substances, a core-shell structure of a mixture of the foregoing substances, a core-shell structure of a gradient mixture of the foregoing substances, or the like. In an implementation of this application, the nanocrystalline particles may be prepared as required, or may be obtained commercially.

In an implementation of this application, the nanocrystalline particles may be quantum dots, and may be specifically red quantum dots, green quantum dots, or blue quantum dots.

In an implementation of this application, a particle size of the nanocrystalline particles may be 5 nm to 20 nm. An appropriate particle size of the nanocrystalline particles is conducive to obtaining good luminescence performance. In some embodiments, the particle size of the nanocrystalline particles may be 5 nm, 6 nm, 7 nm, 8 nm, 9 nm, 10 nm, 11 nm, 12 nm, 13 nm, 14 nm, 15 nm, 16 nm, 17 nm, 18 nm, 19 nm, or 20 nm.

In an implementation of this application, a total mass of the ligand 102 on the surface of the nanocrystalline particles 101 is 1% to 30% of a mass of the nanocrystalline particles 101. To be specific, the total mass of the ligand 102 coordinated to the surface of the nanocrystalline particles 101 is 1% to 30% relative to the mass of the nanocrystalline particles 101. In some embodiments, the total mass of the ligand 102 on the surface of the nanocrystalline particles 101 is 1%, 2%, 5%, 10%, 12%, 15%, 20%, 25%, or 30% of the mass of the nanocrystalline particles 101. Specifically, the total mass of the photosensitive ligand with the structure shown in formula (I) and/or formula (II) on the surface of the nanocrystalline particles 101 is 1% to 30% of the mass of the nanocrystalline particles 101. An appropriate coordination amount of the photosensitive ligand helps the nanocrystalline complex to have high photosensitivity and high thermal stability, and helps well utilize properties of the nanocrystalline particles.

Refer to FIG. 6. The nanocrystalline complex according to embodiments of this application can undergo crosslinking between particles under radiation of ultraviolet light based on the photosensitive ligand with the structure shown in formula (I) and/or formula (II) coordinated to the surface of the nanocrystalline particles. In this case, a patterned film can be directly prepared through exposure with a mask and development with a solvent. Specifically, the nanocrystalline complex in an exposure region undergoes photo-crosslinking under radiation of ultraviolet light to form a film, while the nanocrystalline complex in a non-exposure region does not undergo photo-crosslinking and can be eluted with a solvent, thereby obtaining a patterned film.

An embodiment of this application further provides a method for preparing the foregoing nanocrystalline complex, including:
dispersing nanocrystalline particles whose surface is coordinated with a first ligand in a solvent, adding the photosensitive ligand with the structure shown in formula (I) and/or formula (II), and stirring to undergo a reaction, to obtain ligand-exchanged nanocrystalline particles, that is, to obtain the nanocrystalline complex.

In an implementation of this application, the stirring may be performed at room temperature to undergo the reaction. Time for the stirring to undergo the reaction may be 10 min to 120 min. In an implementation of this application, the first ligand may include a saturated or unsaturated aliphatic carboxylic acid ligand, and may specifically include but is not limited to an oleic acid ligand. In an implementation of this application, the solvent may include but is not limited to one or more of chloroform, chlorobenzene, and ethyl benzoate. In an implementation of this application, a mass of the added photosensitive ligand with the structure shown in formula (I) and/or formula (II) may be equal to a mass of the nanocrystalline particles whose surface is coordinated with the first ligand, that is, the mass of the added photosensitive ligand with the structure shown in formula (I) and/or formula (II) is equal to a total mass of the first ligand and the nanocrystalline particles.

In some implementations of this application, after the reaction with stirring is completed, precipitation may be performed once with excess methanol, and a precipitate is collected to obtain a nanocrystalline complex product in a powder form; or the precipitate may be redissolved in a solvent to obtain a nanocrystalline complex dispersion liquid product. The solvent may include but is not limited to one or more of chloroform, chlorobenzene, and ethyl benzoate.

In an implementation of this application, the photosensitive ligand with the structure shown in formula (I) may be obtained by introducing a target group to hydroxyl-substituted chalcone as a raw material. For example, the photosensitive ligand with the structure shown in formula (I-1) may be obtained through a reaction between hydroxyl-substituted chalcone and diacid, with a preparation process shown in reaction equation (1):

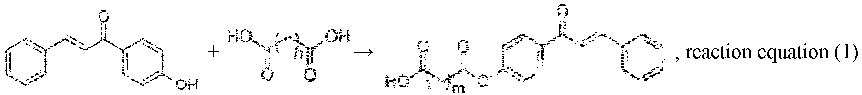

Herein, m may be an integer greater than or equal to 0.

In an implementation of this application, the photosensitive ligand with the structure shown in formula (II) may be obtained by introducing a target group to hydroxyl-substituted coumarin as a raw material. For example, a preparation process of the photosensitive ligand with the structure shown in formula (I-1) may be as follows:

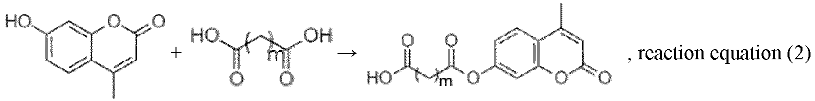

Herein, m may be an integer greater than or equal to 0.

The method for preparing the nanocrystalline complex provided in embodiments of this application has a simple process, which can easily implement quantitative production.

An embodiment of this application further provides a nanocrystalline composition. The nanocrystalline composition includes the foregoing nanocrystalline complex in embodiments of this application and a solvent. In an implementation of this application, the solvent may include but is not limited to one or more of chloroform, chlorobenzene, and ethyl benzoate.

In an implementation of this application, in the nanocrystalline composition, a mass concentration of the nanocrystalline complex may be 1 mg/mL to 100 mg/mL. In some embodiments, the mass concentration of the nanocrystalline complex is 1 mg/mL, 5 mg/mL, 10 mg/mL, 15 mg/mL, 20 mg/mL, 30 mg/mL, 40 mg/mL, 50 mg/mL, 60 mg/mL, 70 mg/mL, 80 mg/mL, 90 mg/mL, or 100 mg/mL. An appropriate concentration of the nanocrystalline complex can help a nanocrystalline film prepared by using the nanocrystalline composition to have good comprehensive performance.

Refer to FIG. 7. An embodiment of this application further provides a method for preparing a patterned film, including:
providing a solution containing the foregoing nanocrystalline complex in embodiments of this application or providing the foregoing nanocrystalline composition on a substrate 11 to form a coating layer 12, and performing exposure and development by using a mask 13, where the nanocrystalline complex in an exposure region undergoes photo-crosslinking to be attached to the substrate 11, and the nanocrystalline complex in a non-exposure region is removed through the development, to form the patterned film 12'.

In an implementation of this application, time for the exposure may be 0.1 seconds to 600 seconds. In some embodiments, the time for the exposure may be, for example, 0.1 seconds, 10 seconds, 15 seconds, 30 seconds, 40 seconds, 50 seconds, 60 seconds, or 100 seconds. In an implementation of this application, the exposure may be performed through radiation of ultraviolet light with a light wavelength of 365 nm and a light intensity of 0.001 mW/cm² to 1000 mW/cm². In some embodiments, the light intensity may be, for example, 0.001 mW/cm², 1 mW/cm², 5 mW/cm², 10 mW/cm², 20 mW/cm², 30 mW/cm², 50 mW/cm², or 100 mW/cm².

The nanocrystalline complex in embodiments of this application has good photosensitivity, which can implement crosslinking with a low radiation dose and short exposure time, to complete preparation of a patterned film, thereby saving energy and time.

In an implementation of this application, the development is development with a solvent, an organic solvent capable of eluting the nanocrystalline complex is used for the development, and the solvent may be, for example, chloroform.

In an implementation of this application, after the coating layer is formed, annealing may be performed before the exposure and development. Since the nanocrystalline complex in embodiments of this application has low heatsensitive crosslinking activity, crosslinking of the coating layer of the nanocrystalline complex during annealing can be avoided, which affects a fine patterning result.

According to the method for preparing the patterned film in embodiments of this application, good crosslinking effect can be achieved with short exposure time, and only development with a solvent is required, which makes a process simple. This can not only save energy and time, but also obtain a pattern with high resolution.

An embodiment of this application further provides a patterned film formed through crosslinking of the foregoing nanocrystalline complex in embodiments of this application; or a patterned film prepared by the foregoing preparation method in embodiments of this application. The patterned film can be used to prepare a pattern with high resolution, for example, obtain a sub-pixel size less than 10 micrometers or even less than 3 micrometers.

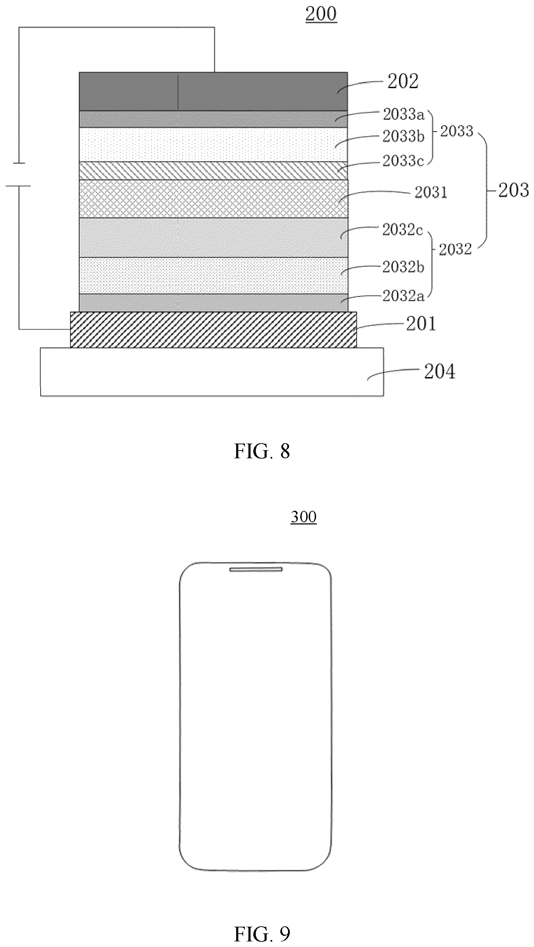

Refer to FIG. 8. An embodiment of this application further provides an electronic component 200. The electronic component 200 includes a first electrode 201 and a second electrode 202 that are opposite to each other, and a functional layer 203 between the first electrode 201 and the second electrode 202. The functional layer 203 includes a crosslinking product of the nanocrystalline complex 100 in embodiments of this application; or the functional layer 203 includes the patterned film in embodiments of this application.

In an implementation of this application, the electronic component 200 may include but is not limited to any one of an LED, a QLED, a mini-LED, a micro-LED, a nano-LED, and a QD-OLED. In an implementation of this application, the electronic component 200 may be an upright component, or may be an inverted component.

In an implementation of this application, the first electrode 201 and the second electrode 202 are respectively an anode and a cathode. Materials for the anode and the cathode are conductive materials, and may be independently selected from conductive metals, conductive metal oxides, conductive polymers, and the like. The conductive metals may include one or more of metal elementary substances such as magnesium (Mg), aluminum (Al), gold (Au), silver (Ag), platinum (Pt), and palladium (Pd), and alloys thereof. The conductive metal oxides include but are not limited to one or more of indium tin oxide (ITO), indium zinc oxide (IZO), aluminum-doped zinc oxide (AZO), fluorinedoped tin dioxide (FTO), phosphorus-doped tin dioxide (PTO), and the like. The conductive polymers include but are not limited to polythiophene, polypyrrole, polyphenylamine, and the like.

In an implementation of this application, the functional layer 203 includes a light-emitting layer 2031. The light-emitting layer 2031 includes a crosslinking product of the foregoing nanocrystalline complex 100 in embodiments of this application; or the functional layer 203 includes the foregoing patterned film in embodiments of this application.

In an implementation of this application, the functional layer 203 further includes a first carrier transport layer 2032 between the first electrode 201 and the light-emitting layer 2031, and a second carrier transport layer 2033 between the second electrode 202 and the light-emitting layer 2031. For example, the first electrode 201 is an anode, and the second electrode 202 is a cathode. The first carrier transport layer 2032 may include one or more of a hole injection layer 2032a, a hole transport layer 2032b, and an electron blocking layer 2032c that are between the anode 201 and the light-emitting layer 2031. The hole injection layer 2032a is located between the anode 201 and the hole transport layer 2032b, and the electron blocking layer 2032c is located between the light-emitting layer 2031 and the hole transport layer 2032b. The second carrier transport layer 2033 may include one or more of an electron injection layer 2033a, an electron transport layer 2033b, and a hole blocking layer 2033c that are between the cathode 202 and the light-emitting layer 2031. The electron injection layer 2033a is located between the cathode 202 and the electron transport layer 2033b, and the hole blocking layer 2033c is located between the cathode 202 and the hole transport layer 2032b. In some embodiments, as shown in FIG. 8, the electronic component 200 includes the anode 201, the hole injection layer 2032a, the hole transport layer 2032b, the electron blocking layer 2032c, the light-emitting layer 2031, the hole blocking layer 2033c, the electron transport layer 2033b, and the electron injection layer 2033a that are sequentially provided. It should be noted that not all of the layers of the functional layer 203 are necessary, but the light-emitting layer 2031 is necessary. For example, in a direction from the anode 201 to the cathode 202, the functional layer 203 may alternatively sequentially include a stacked structure of "light-emitting layer 2031/electron transport layer 2033b", or include a stacked structure of "light-emitting layer 2031/electron injection layer 2033a", or include a stacked structure of "hole injection layer 2032a/light-emitting layer 2031/electron transport layer 2033b", or include a stacked structure of "hole injection layer 2032a/light-emitting layer 2031/electron injection layer 2033a", or include a stacked structure of "hole transport layer 2032b/light-emitting layer 2031/electron transport layer 2033b", or include a stacked structure of "hole injection layer 2032a/hole transport layer 2032b/light-emitting layer 2031/electron transport layer 2033b", or include a stacked structure of "hole injection layer 2032a/hole transport layer 2032b or electron blocking layer 2032c/light-emitting layer 2031/hole blocking layer 2033c or electron transport layer 2033b/electron injection layer 2033a", or include a stacked structure of "hole injection layer 2032a/hole transport layer 2032b/electron blocking layer 2032c/light-emitting layer 2031/hole blocking layer 2033c or electron transport layer 2033b/electron injection layer 2033a", or the like. Herein, "/" indicates a boundary of the layers. In this application, a thickness of each layer is not specifically limited, and may be determined by a person skilled in the art based on an actual requirement. A material of each layer is a conventional choice in the art, and is not specifically limited in this application.

In some implementations, the electronic component 200 may further include a base plate 204 (as shown in FIG. 8). The base plate 204 may be located on a side that is of the anode 201 and that is away from the functional layer 203 (as shown in FIG. 8), or the base plate 204 may be located on a side that is of the cathode 202 and that is away from the functional layer 203. In other words, the electronic component 200 may be a bottom emission component, or may be a top emission component. The base plate 204 may be used as a support part of the entire electronic component 200, and may be made of quartz, glass, monatomic silicon, metal, plastic, or the like. In some implementations, the base plate 204 is glass or plastic transparent to light. A shape of the base plate 204 may be determined based on a specific application scenario, for example, may be a plate, a film, or a sheet. A thickness of the base plate 204 is not specifically limited. The base plate 204 may include an active matrix (active matrix) or passive matrix (passive matrix) drive circuit.

In this application, preparation processes of the anode 201, the cathode 202, and each layer of the functional layer 203 are not specifically limited, and may be performed by a physical vapor deposition method, a chemical vapor deposition method, a coating method, or the like. The physical vapor deposition method may include one or more of a vacuum evaporation method (such as resistance evaporation, electron beam evaporation, and pulsed laser deposition), a sputtering method (such as magnetron sputtering), and the like. The coating method may include solution spin coating, dip coating, blade coating, spray coating, roll coating, inkjet printing, screen printing, and the like. Usually, the anode 201 and the cathode 202 may be prepared by a vacuum evaporation method, and each layer of the functional layer 203 may be prepared by a vacuum evaporation method or a coating method. The manufacture of the electronic component 200 shown in FIG. 8 is used as an example. The anode 201 may be first formed on the base plate 204, then the functional layer 203 including the light-emitting layer 2031 is sequentially formed on the anode 201, and then the cathode 202 is formed on the functional layer 203. In another implementation of this application, the cathode 202 and the functional layer 203 including the light-emitting layer 2031 may be sequentially formed on the base plate 204, and then the anode 201 is formed on the functional layer 203.

As an example, a material for the hole injection layer includes but is not limited to an aniline conductive polymer, a polythiophene conductive polymer, and the like. A material for the hole transport layer includes but is not limited to an arylamine organic material (for example, TFB), and the like. A material for the electron transport layer may include but is not limited to an alkaline earth metal oxide (for example, zinc oxide), and the like. In addition, in this application, a thickness of each layer is not specifically limited, and may be determined by a person skilled in the art based on an actual requirement.

In some embodiments, a process of manufacturing a quantum dot light-emitting device QLED may be as follows:
preparing a hole injection layer on a cleaned ITO anode base plate;
preparing a hole transport layer on the hole injection layer;
preparing red, green, and blue three-color patterned quantum dot film layers in sequence on the hole transport layer;
preparing an electron injection layer on the red, green, and blue three-color patterned quantum dot film layers; and
preparing a metal electrode on the electron injection layer.

In a specific embodiment of this application, a process of manufacturing a quantum dot light-emitting device QLED may be as follows:
(1) A clean ITO base plate is subjected to ultrasonic treatment with deionized water and ethanol ultrasound sequentially for 10 min. The cleaned ITO base plate is spin-coated with an aqueous solution of poly(3,4-ethylenedioxythiophene)-polystyrenesulfonic acid (pedot:pss) at a rotational speed of 2500 rpm and an acceleration of 800 rpm for 30s, and then heated in the air to 150°C and kept for 30 min, to obtain a pedot:pss film layer.
(2) The pedot:pss film layer is spin-coated with 6 mg/mL of crosslinkable TFB (poly[(9,9-fluorenyl-2,7-diyl)-alt-(4,4'-(N-(4-sec-butylphenyl))diphenylamine)]) in chlorobenzene solution at a rotational speed of 2800 rpm and an acceleration of 900 rpm for 30s, and then annealed at 190°C in a nitrogen atmosphere for 30 min, to obtain a TFB film layer.
(3) The TFB film layer is spin-coated with a red quantum dot solution in embodiments of this application at a rotational speed of 2500 rpm and an acceleration of 800 rpm for 30s, to obtain a quantum dot film layer. Then, the quantum dot film layer is irradiated with a 365 nm LED flat panel light source (with an energy density of about 30 mW/cm²) using a mask corresponding to red sub-pixels, to undergo crosslinking for 15s to 60s.
(4) The quantum dot film layer is spin-coated with a chloroform solution at a rotational speed of 2500 rpm and an acceleration of 800 rpm for 30s to undergo development to remove an unexposed part of the red quantum dot layer.
(5) Exposure and development are performed in sequence according to step (3) and step (4) to prepare patterned films of green quantum dots and blue quantum dots.
(6) The red, green, and blue quantum dot film layers are spin-coated with 30 mg/mL of ZnO solution at a rotational speed of 4000 rpm and an acceleration of 4000 rpm for 30s.
(7) Finally, a 100 nm silver electrode is subjected to evaporation at a rate of 0.1 nm/s.

As known to a person skilled in the art, red, green, and blue components in a top emission component may require different microcavity structures, and hole transport layers (for example, with TFB materials) and/or electron transport layers (for example, ZnO or other metal oxides with an electron transport capability) of different thicknesses may be prepared for red, green, and blue sub-pixels as required. In addition, a sequence of preparing the patterned films of the red, green, and blue quantum dots may be changed as required.

Refer to FIG. 9. An embodiment of this application further provides a display apparatus 300. The display apparatus 300 includes the foregoing electronic component 200 in embodiments of this application.

In an implementation of this application, the display apparatus 300 may be a visual display apparatus in any product or component with a display function, such as a mobile phone, a tablet computer, a notebook computer, a wearable device (such as a smartwatch or a smart band), a television, a digital camera, a camcorder, a player, a micro display device (such as smart glasses, a virtual reality (Virtual Reality, VR) device, or an augmented reality (Augmented Reality, AR) device), a telephone set, a printer, transportation, a household appliance, an advertising board, an information board, or a central control screen of a vehicle.

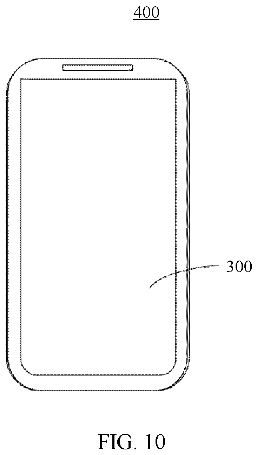

Refer to FIG. 10. An embodiment of this application further provides an electronic device 400. The electronic device 400 includes the foregoing display apparatus 300 in embodiments of this application. The electronic device 400 may be any electronic product with a display function, such as a mobile phone, a tablet computer, a notebook computer, a wearable device (such as a smartwatch or a smart band), a television, a digital camera, a camcorder, a player, a micro display device (such as smart glasses, a virtual reality (Virtual Reality, VR) device, or an augmented reality (Augmented Reality, AR) device), a telephone set, a printer, transportation, a household appliance, an advertising board, an information board, or a central control screen of a vehicle.

The following further describes embodiments of this application by using a plurality of embodiments.

### Embodiment 1

### Synthesis of a photosensitive ligand ChalBen:

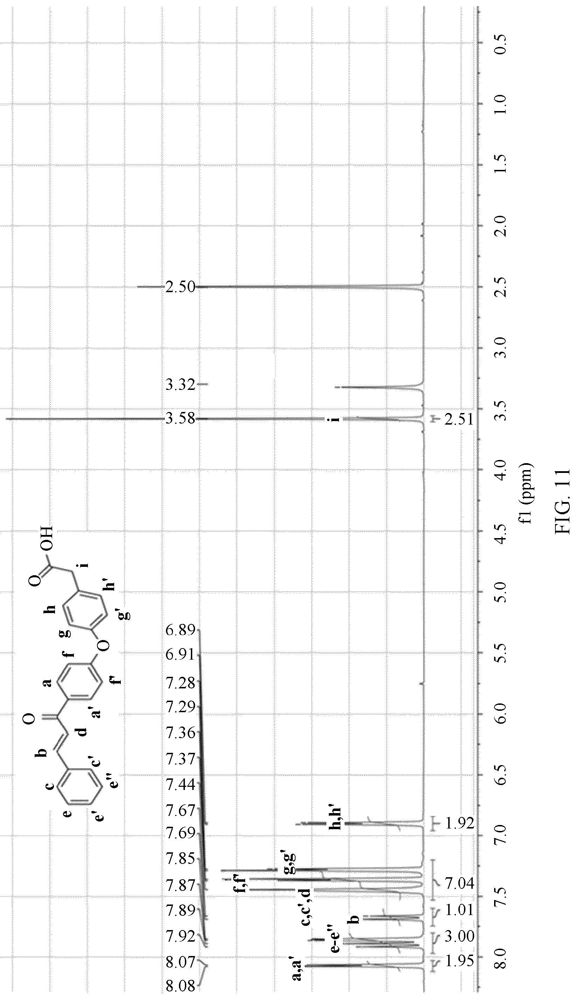

Acid reactants p-chlorophenylacetic acid and K₂CO₃ are added to a hydroxyl-substituted chalcone in DMF (N,N-dimethylformamide) solution at room temperature, refluxed for 1.5 h, and then cooled down to room temperature to obtain a reaction solution. Deionized water us added to the reaction solution, and then hydrochloric acid is added to adjust pH to acidic, to obtain a mixed system. Then, dichloromethane DCM is added to the mixed system to extract a product, and finally an organic phase is dried with magnesium sulfate MgSO₄. The organic phase is concentrated and then purified by silica gel column chromatography with n-hexane and ethyl acetate in a volume ratio of 2:3, to obtain the photosensitive ligand ChalBen. FIG. 11 is a nuclear magnetic resonance spectrum of the photosensitive ligand ChalBen prepared according to Embodiment 1 of this application. The foregoing reaction process is shown in formula (1):

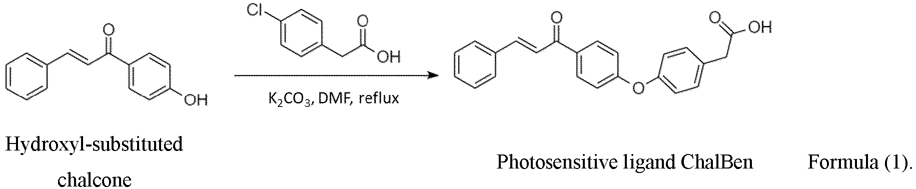

### Embodiment 2

### Synthesis of a photosensitive ligand ChalC5

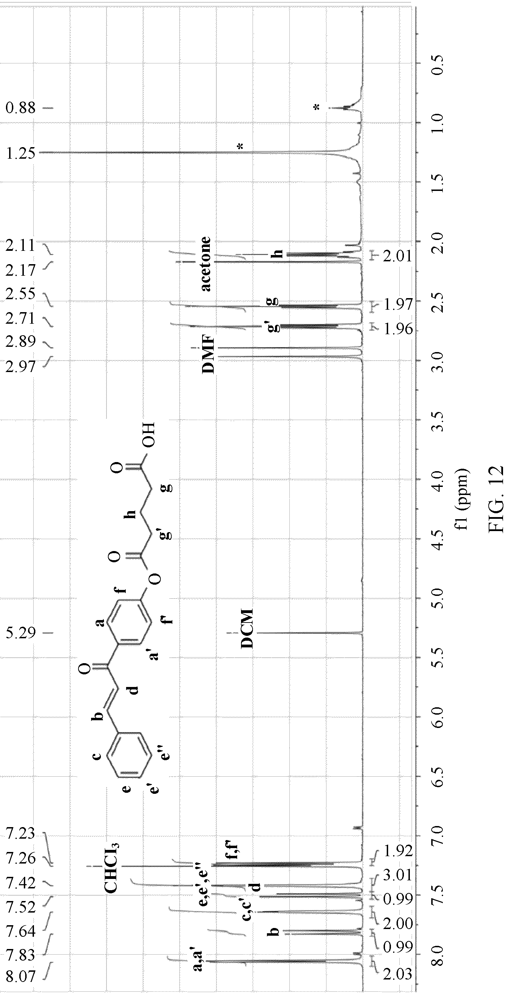

K₂CO₃ and glutaric anhydride are added to a hydroxyl-substituted chalcone in DMF solution to undergo a reaction kept at 50°C for about 2 h. After the reaction, 2 mol/L of hydrochloric acid solution is added to the mixed system to adjust to pH ≈ 2-3. Then, dichloromethane DCM is added to the mixed system to extract a product, and finally an organic phase is dried with magnesium sulfate MgSO₄. The organic phase is concentrated and then purified by silica gel column chromatography with dichloromethane and ethyl acetate in a volume ratio of 10:1, to obtain the photosensitive ligand ChalC5. FIG. 12 is a nuclear magnetic resonance spectrum of the photosensitive ligand ChalC5 prepared according to Embodiment 2 of this application. The reaction process is shown in formula (2):

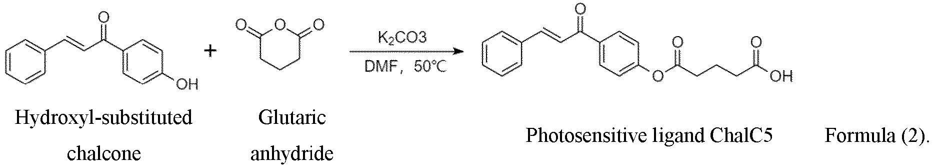

### Embodiment 3

### Synthesis of a photosensitive ligand ChalC8

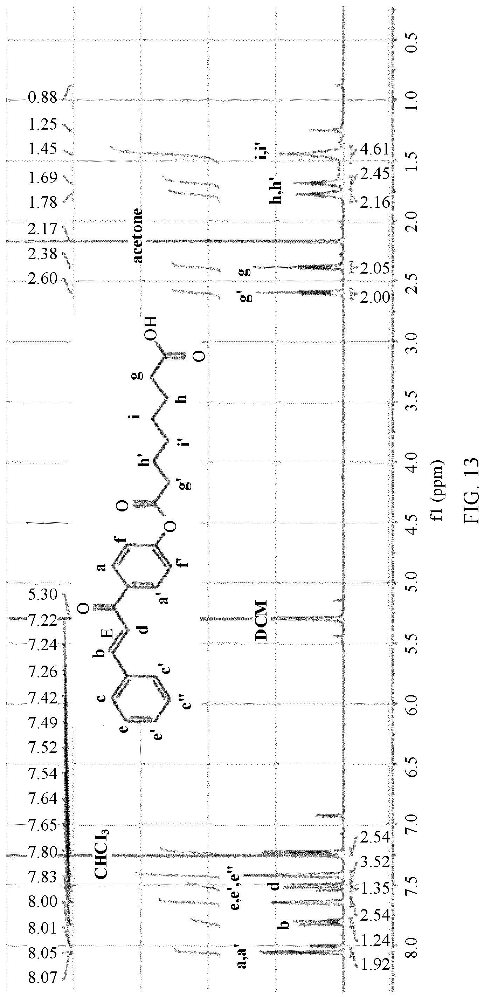

Octanedioic acid, acetonitrile (ACN), carbodiimide (EDAC), and 4-dimethylaminopyridine (DMAP) solution are mixed and stirred at 0°C for 1 h, to obtain a mixed system. Hydroxyl-substituted chalcone is added to the obtained mixed system at room temperature to undergo a reaction kept at 42°C for 50 min. Rotary evaporation is performed to remove acetonitrile. The remaining mixture is redissolved in dichloromethane and washed with 2 mol/L HCl, saturated NaCl solution, and deionized water in sequence. Silica gel column chromatography is performed for purification with dichloromethane and ethyl acetate in a volume ratio of 10:1, to obtain the photosensitive ligand ChalC8. FIG. 13 is a nuclear magnetic resonance spectrum of the photosensitive ligand ChalC8 prepared according to Embodiment 3 of this application. The reaction process is shown in formula (3):

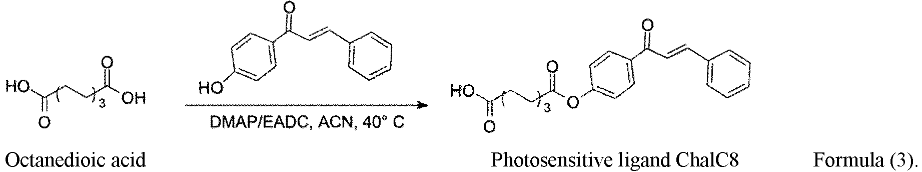

### Embodiment 4

### Synthesis of a photosensitive ligand ChalC13

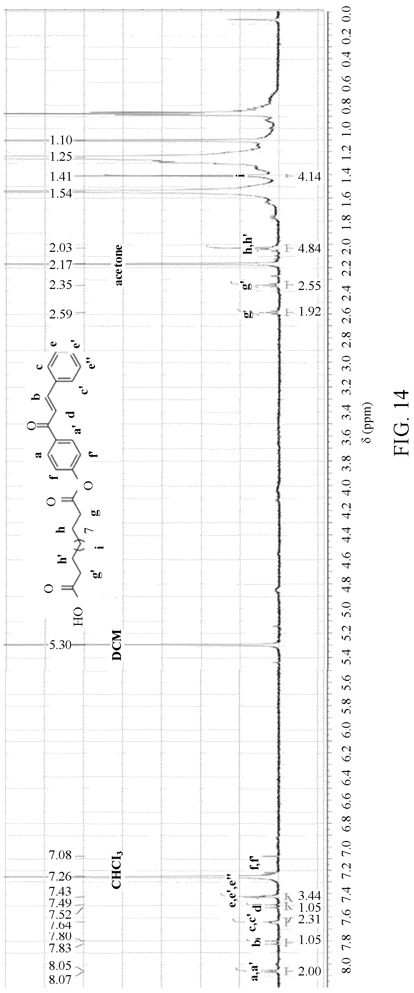

Dodecanedioic acid, acetonitrile (ACN), carbodiimide (EDAC), and 4-dimethylaminopyridine (DMAP) solution are mixed and stirred at 0°C for 1 h, to obtain a mixed system. Hydroxyl-substituted chalcone is added to the obtained mixed system at room temperature to undergo a reaction kept at 42°C for 1 h. Rotary evaporation is performed to remove acetonitrile. The remaining mixture is redissolved in dichloromethane and washed with 2 mol/L HCl, saturated NaCl solution, and deionized water in sequence. Silica gel column chromatography is performed for purification with dichloromethane and ethyl acetate in a volume ratio of 10:1, to obtain the photosensitive ligand ChalC8. FIG. 14 is a nuclear magnetic resonance spectrum of the photosensitive ligand ChalC13 prepared according to Embodiment 4 of this application. The reaction process is shown in formula (4):

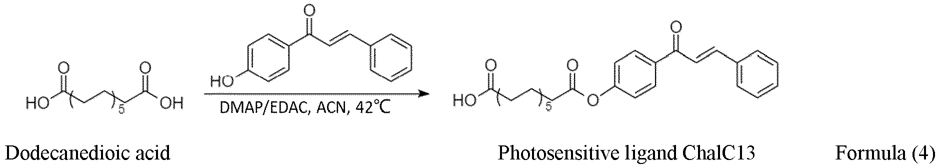

### Embodiment 5

### Synthesis of a photosensitive ligand CouC2

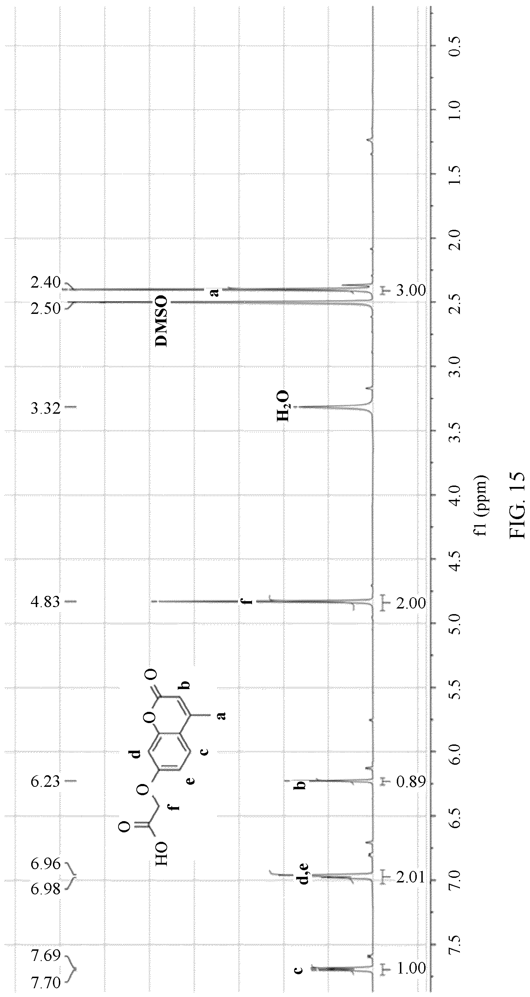

Chloroacetic acid and potassium carbonate K₂CO₃ are added to a hydroxyl-substituted coumarin in DMF solution at room temperature, and refluxed at 155°C for 1 h. Then, deionized water is added, 2 mol/L HCl is added to adjust pH to acidic, filtered, and washed with water. The obtained solid is evaporated and dried to obtain the photosensitive ligand CouC2. FIG. 15 is a nuclear magnetic resonance spectrum of the photosensitive ligand CouC2 prepared according to Embodiment 5 of this application. The reaction process is shown in formula (5):

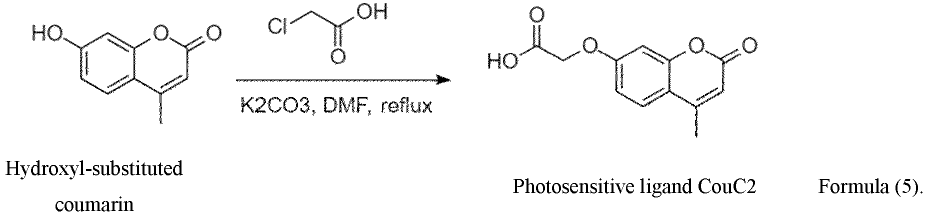

### Embodiment 6

### Synthesis of a photosensitive ligand CouC8

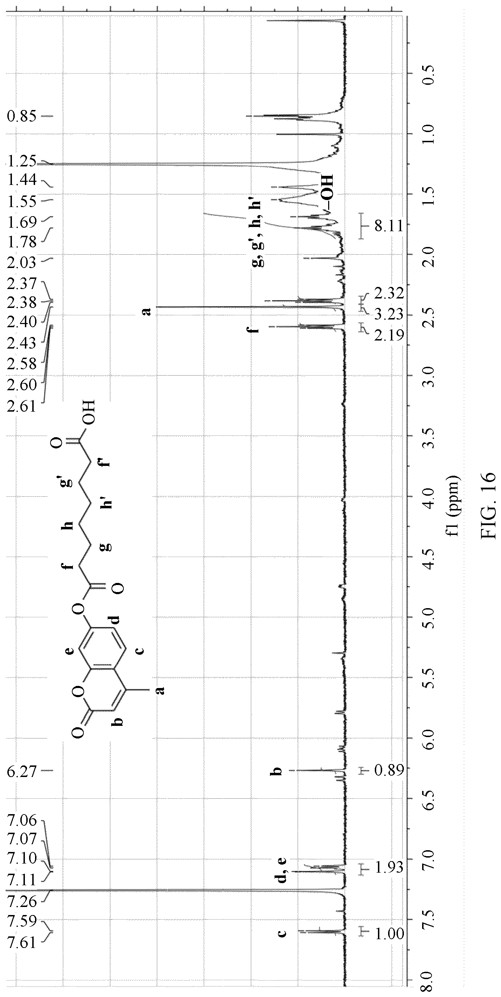

Octanedioic acid, acetonitrile (ACN), carbodiimide (EDAC), and 4-dimethylaminopyridine (DMAP) solution are mixed and stirred at 0°C for 1 h, to obtain a mixed system. Hydroxyl-substituted coumarin is dissolved in acetonitrile and added to the obtained mixed system at room temperature to undergo a reaction kept at 40°C to 45°C for 45 min. Rotary evaporation is performed to remove acetonitrile. The remaining mixture is redissolved in chloroform and washed with 2 mol/L HCl, saturated NaCl solution, and deionized water in sequence. An organic phase is dried with magnesium sulfate MgSO₄, and then the solvent chloroform is removed. Finally, recrystallization is performed with ethanol, filtration is performed to retain a liquid phase, and ethanol is removed to obtain the photosensitive ligand ChalC8. FIG. 16 is a nuclear magnetic resonance spectrum of the photosensitive ligand CouC8 prepared according to Embodiment 6 of this application. The reaction process is shown in formula (6):

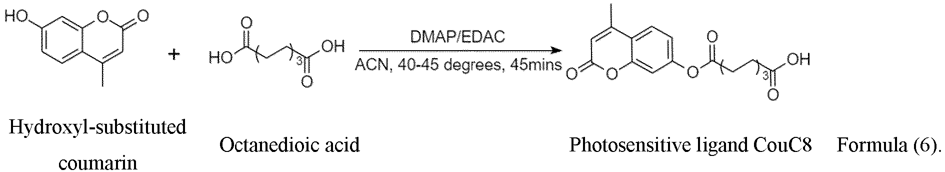

### Embodiment 7

### Synthesis of a photosensitive ligand CouC13

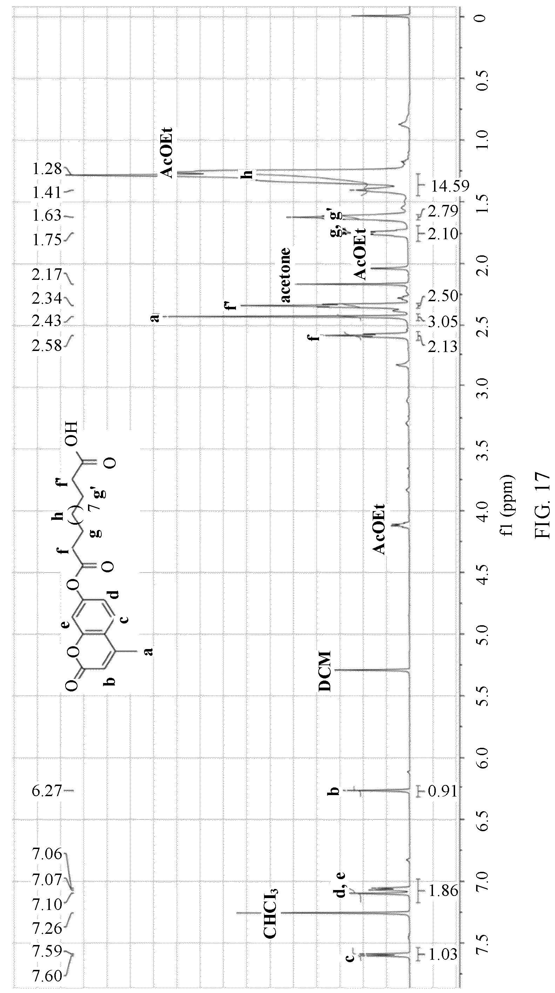

Tridecanedioic acid, acetonitrile (ACN), carbodiimide (EDAC), and 4-dimethylaminopyridine (DMAP) solution are mixed and stirred at 0°C for 1 h, to obtain a mixed system. Hydroxyl-substituted coumarin is dissolved in acetonitrile and added to the obtained mixed system at room temperature to undergo a reaction kept at 45°C for 2 h. Rotary evaporation is performed to remove acetonitrile. The remaining mixture is redissolved in chloroform and washed with 2 mol/L HCl, saturated NaCl solution, and deionized water in sequence. An organic phase is dried with MgSO₄, and then the solvent chloroform is removed. Finally, recrystallization is performed with ethanol, filtration is performed to retain a liquid phase, and ethanol is removed to obtain a white solid product, that is, to obtain the photosensitive ligand ChalC13. FIG. 17 is a nuclear magnetic resonance spectrum of the photosensitive ligand CouC13 prepared according to Embodiment 7 of this application. The reaction process is shown in formula (7):

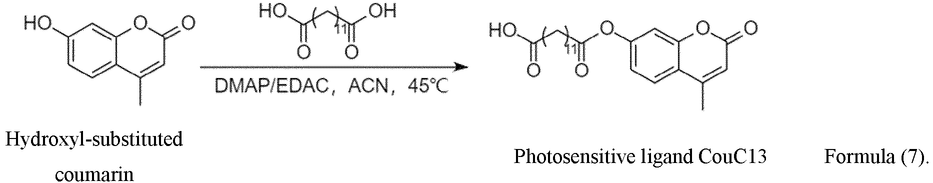

Green quantum dots are used as an example. Synthesis of the green quantum dots includes the following steps:
(1) 5 mmol of Se powder and 5 mmol of S powder are dissolved in 5 mL of trioctylphosphine (TOP) solution to obtain a (Se+S)-TOP precursor.
(2) 0.14 mmol of cadmium acetate, 3.41 mmol of zinc oxide, and 7 mL of oleic acid are added in a 50 mL three-necked flask, and heated under nitrogen protection to 150°C for 30 min to remove acetic acid and water. 15 mL of octadecene (ODE) is added, and the reaction system is heated to 310°C. 2 mL of (Se+S)-TOP precursor is added, and the three-necked flask is cooled down to 300°C, to carry out a reaction for 15 min, to obtain CdZnSeS/ZnS green quantum dots.
(3) An excess mixed solution of methanol and acetone (in a volume ratio of 3:1) is added to precipitate the quantum dots, and centrifuged at 8000 rpm for 5 min. The precipitate is dissolved in 5 mL of octane solution, precipitated again with 15 mL of acetone, and centrifuged at 8000 rpm for 5 min. This step is repeated once.
(4) The obtained quantum dot precipitate is prepared into a 50 mg/mL chloroform solution for use.

### Quantum dot ligand exchange:

For example, an oleic acid ligand is exchanged into the photosensitive ligand ChalC8 prepared in Embodiment 3. 1 mL of the 50 mg/mL quantum dots in chloroform solution is taken, and the photosensitive ligand ChalC8 (50 mg) of the equal mass as the quantum dots is added and stirred at room temperature for 30 min, to obtain ligand-exchanged quantum dots, that is, obtain quantum dots whose surface is coordinated with the photosensitive ligand ChalC8, that is, a quantum dot complex. The quantum dot complex is precipitated once with excess methanol. The precipitate is redissolved in a chloroform solution and prepared into a 7.5 mg/mL quantum dot solution for spin coating.

A patterned quantum dot film is prepared by using the obtained quantum dot solution, and evaluated for photo-crosslinking effect and thermal crosslinking performance:
Preparation of a patterned quantum dot film:
(1) Preparation of a quantum dot film: A clean glass sheet is spin-coated with a 30 mg/mL ZnO ethanol solution at a rotational speed of 2800 rpm and an acceleration of 900 rpm for 30s. After spin coating, annealing is performed at 150°C in a nitrogen atmosphere for 30 min to obtain a ZnO film. Then, the ZnO film is spin-coated with the obtained quantum dot solution at a rotational speed of 2500 rpm and an acceleration of 800 rpm for 30s.
(2) A quantum dot film sample obtained through spin coating is annealed at 100°C for 10 min.
(3) Exposure: The quantum dot film layer is irradiated with a 365 nm LED flat panel light source (with an energy density of about 30 mW/cm²) to undergo crosslinking for 15s to 60s.
(4) Development: The entire glass sheet with the quantum dot film layer is immersed in a chloroform solution, and left to stand for 1 min. The quantum dot complex in an exposure region undergoes photo-crosslinking under radiation of ultraviolet light to form a film, while the quantum dot complex in a non-exposure region does not undergo photo-crosslinking and can be eluted with a solvent, thereby obtaining a patterned quantum dot film.

### Evaluation for photo-crosslinking effect and thermal crosslinking performance:

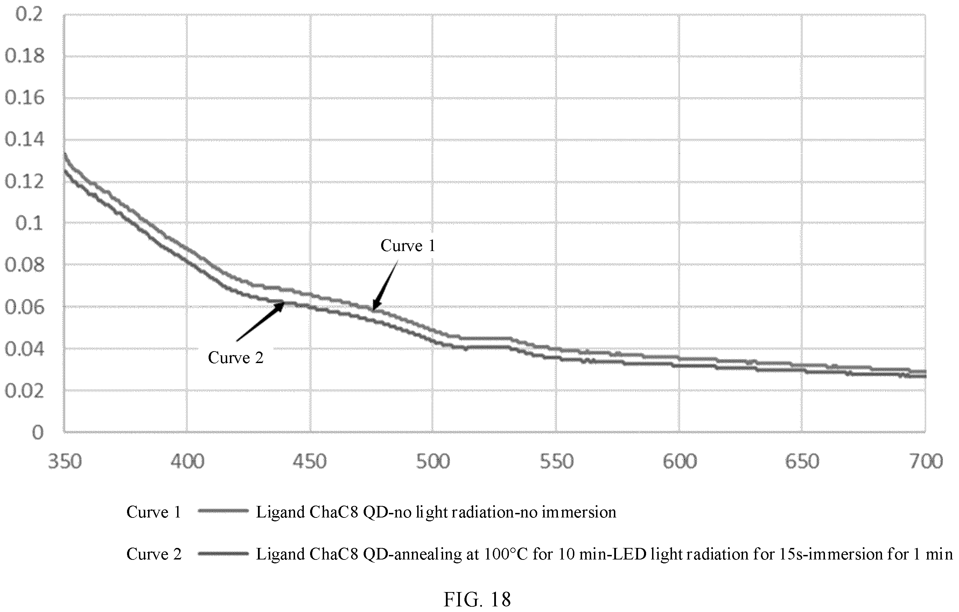

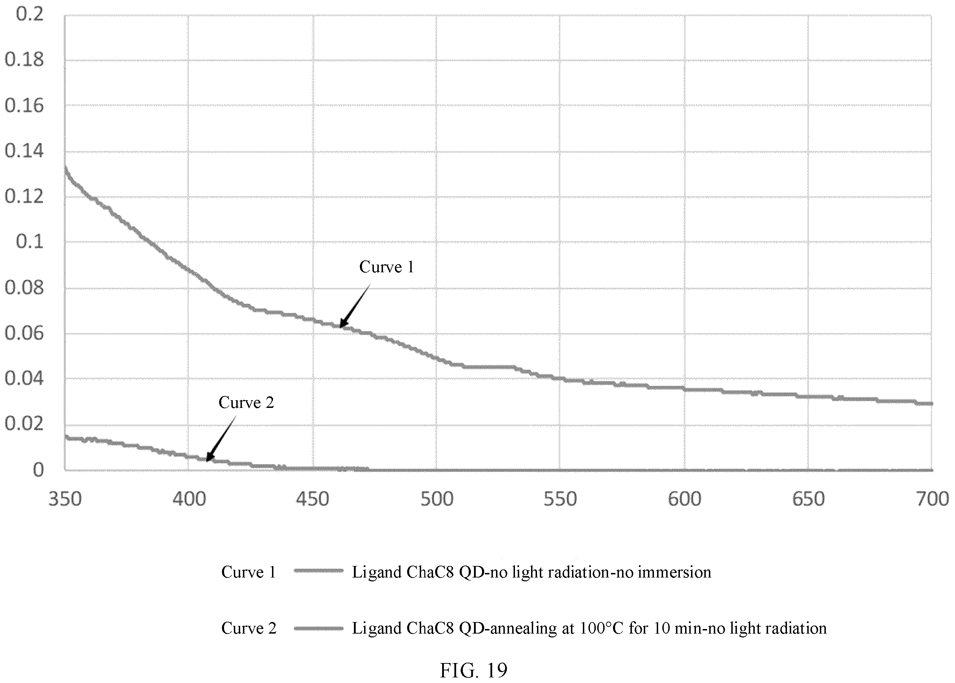

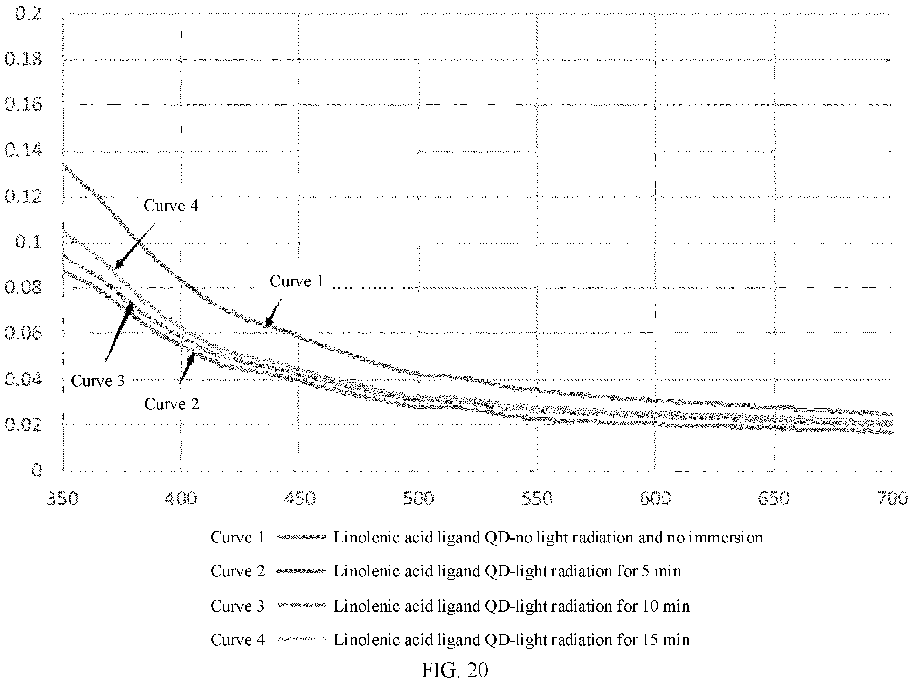

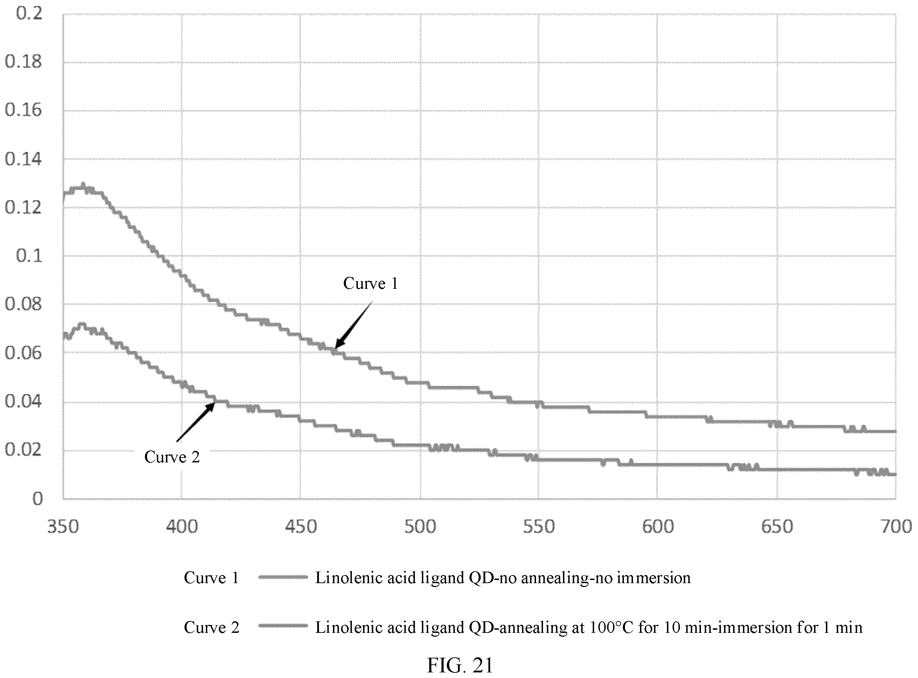

UV-VIS (ultraviolet-visible) absorption curves of the quantum dot film sample at different stages are measured. The results are shown in FIG. 18 and FIG. 19. Quantum dots whose surface is coordinated with a linolenic acid ligand are used as a comparative example. UV-VIS absorption curves of the quantum dot film sample at different stages are measured. The results are shown in FIG. 20 and FIG. 21. Elution with a developing solvent is immersion in a chloroform solution for 1 min.

In FIG. 18, curve 1 is a UV-VIS absorption curve of the quantum dot film whose surface is coordinated with the ligand ChalC8 without photo-crosslinking and elution with a developing solvent (immersion in a chloroform solution for 1 min) (that is, an experimental result of a blank control without any treatment), and curve 2 is a UV-VIS absorption curve of the quantum dot film whose surface is coordinated with the ligand ChalC8 after annealing at 100°C for 10 min, photo-crosslinking for 15s, and elution with a developing solvent. It can be learned from FIG. 18 that, the quantum dots whose surface is coordinated with the ligand ChalC8 after annealing at 100°C for 10 min, photo-crosslinking for 15s, and elution with a developing solvent have a retention rate greater than 95%, indicating that the ligand ChalC8 has a high photo-crosslinking degree. In FIG. 20, curve 1 is a UV-VIS absorption curve of the quantum dot film whose surface is coordinated with the linolenic acid ligand without photo-crosslinking and elution with a developing solvent (immersion in a chloroform solution for 1 min) (that is, an experimental result of a blank control without any treatment), and curve 2, curve 3, and curve 4 are UV-VIS absorption curves of the quantum dot film whose surface is coordinated with the linolenic acid ligand respectively after photo-crosslinking for 5 min, 10 min, and 15 min, and elution with a developing solvent. It can be learned from FIG. 20 that, under the same conditions of elution with a developing solvent, the quantum dots whose surface is coordinated with the linolenic acid ligand after photo-crosslinking for 5 min to 15 min have a retention rate less than 80%. Compared with the comparative example, the quantum dots whose surface is coordinated with the ligand ChalC8 in this embodiment of this application can undergo full crosslinking under light irradiation in shorter time, and have good photo-crosslinking effect. If the absorbance on the UV-VIS absorption curve of the quantum dot film sample after exposure-development is significantly lower than that of the quantum dot film sample before exposure-development, the quantum dot complex has poor photo-crosslinking effect, and consequently the quantum dot complex is washed off by the developing solvent. If the absorbance on the UV-VIS absorption curve of the quantum dot film sample after exposure-development is significantly higher than that of the quantum dot film sample before exposure-development, photo-crosslinking effect is good.

In FIG. 19, curve 1 is a UV-VIS absorption curve of the quantum dot film whose surface is coordinated with the ligand ChalC8 without photo-crosslinking, annealing, and elution with a developing solvent (immersion in a chloroform solution for 1 min) (that is, an experimental result of a blank control without any treatment), and curve 2 is a UV-VIS absorption curve of the quantum dot film whose surface is coordinated with the ligand ChalC8 only after annealing at 100°C for 10 min and then elution with a developing solvent, without photo-crosslinking. It can be learned from FIG. 19 that, through annealing at 100°C for 10 min, the ligand ChalC8 is not likely to undergo crosslinking spontaneously, and has a residual amount less than 15% after elution, indicating that the ligand ChalC8 has a low thermal crosslinking degree. In FIG. 21, curve 1 is a UV-VIS absorption curve of the quantum dot film whose surface is coordinated with the linolenic acid ligand without photo-crosslinking, annealing, and elution with a developing solvent (immersion in a chloroform solution for 1 min) (that is, an experimental result of a blank control without any treatment), and curve 2 is a UV-VIS absorption curve of the quantum dot film whose surface is coordinated with the linolenic acid ligand after annealing at 100°C for 10 min and then elution with a developing solvent, without photo-crosslinking. It can be learned from FIG. 21 that, through annealing at 100°C for 10 min, a residual amount of the quantum dots whose surface is coordinated with the linolenic acid ligand in the comparative example reaches 50%, indicating that the linolenic acid ligand has a high thermal crosslinking degree.

The photosensitive ligands in Embodiment 1, Embodiment 2, and Embodiment 4 to Embodiment 7 are prepared into quantum dot solutions by the same method above, and then prepared into patterned quantum dot films. It can be learned through detection that each of the photosensitive ligands in Embodiment 1, Embodiment 2, and Embodiment 4 to Embodiment 7 can obtain a retention rate greater than 90% after photo-crosslinking for 15s to 60s, and a residual rate after elution lower than 20% under the annealing conditions, indicating that the nanocrystalline complexes prepared with the photosensitive ligands in Embodiment 1, Embodiment 2, and Embodiment 4 to Embodiment 7 have high photo-crosslinking activity and low thermal crosslinking activity.

It should be understood that "first", "second", and various numbers in this specification are merely used for differentiation for ease of description, but are not intended to limit the scope of this application.

In this application, "and/or" describes an association relationship between associated objects, and indicates that three relationships may exist. For example, A and/or B may indicate that: only A exists, both A and B exist, and only B exists, where A and B may be singular or plural. The character "/" generally indicates an "or" relationship between the associated objects.

In this application, "at least one" means one or more, and "a plurality of" means two or more. "At least one of the following items (pieces)" or a similar expression thereof means any combination of these items, including any combination of singular items (pieces) or plural items (pieces). For example, "at least one of a, b, or c" or "at least one of a, b, and c" may indicate: a, b, c, a-b (namely, a and b), a-c, b-c, or a-b-c, where a, b, and c may be singular or plural.

It should be understood that sequence numbers of the foregoing processes do not mean an execution sequence in various embodiments of this application. Some or all of the steps may be performed in parallel or in sequence. The execution sequence of the processes should be determined based on functions and internal logic of the processes, and should not be construed as any limitation on the implementation processes of embodiments of this application.

## Claims

1. A nanocrystalline complex, wherein the nanocrystalline complex comprises nanocrystalline particles and a ligand coordinated to a surface of the nanocrystalline particles, and the ligand comprises a photosensitive ligand with a structure shown in formula (I) and/or formula (II), 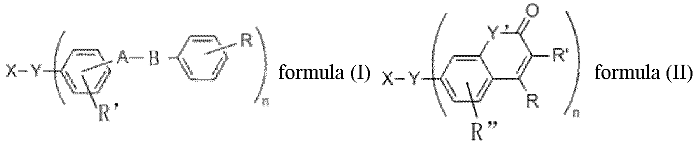
in formula (I), X is a coordinating group coordinated to the nanocrystalline particles, Y is a linking group, one of A and B is -C=O-, the other is a group containing a carbon-carbon double bond, and R and R' are independently one of a hydrogen atom, a halogen atom, a nitro group, a hydroxyl group, a substituted or unsubstituted alkyl group, a substituted or unsubstituted alkoxy group, a substituted or unsubstituted alkyl carbonyl group, a substituted or unsubstituted cycloalkyl group, a substituted or unsubstituted aryl group, and a substituted or unsubstituted aryloxy group;
in formula (II), X is a coordinating group coordinated to the nanocrystalline particles, Y and Y' are linking groups, and R, R', and R" are independently one of a hydrogen atom, a halogen atom, a nitro group, a hydroxyl group, a substituted or unsubstituted alkyl group, a substituted or unsubstituted alkoxy group, a substituted or unsubstituted alkyl carbonyl group, a substituted or unsubstituted cycloalkyl group, a substituted or unsubstituted aryl group, and a substituted or unsubstituted aryloxy group; and
n is 1, 2, or 3.

2. The nanocrystalline complex according to claim 1, wherein the coordinating group comprises any one of a carboxyl group, an amino group, a phosphate group, a phospholipid group, and a mercapto group.

3. The nanocrystalline complex according to claim 1 or 2, wherein the linking group Y comprises one or more of a substituted or unsubstituted alkylene group, a substituted or unsubstituted arylene group, a substituted or unsubstituted arylene alkyl group, a substituted or unsubstituted alkylene aryl group, a substituted or unsubstituted alkyleneoxy group, a substituted or unsubstituted aryleneoxy group, a substituted or unsubstituted alkylene aryloxy group, a substituted or unsubstituted arylene alkoxy group, a carbonyl-containing group, an ester-containing and/or ether-oxygen bond-containing group, and an imino-containing group.

4. The nanocrystalline complex according to claim 3, wherein the carbonyl-containing group comprises any one of -C(=O)- and -R₁-C(=O)-; the ester-containing and/or ether-oxygen bond-containing group comprises any one of - R₂-C(=O)-O-, 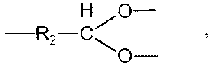 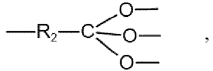 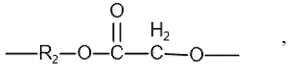 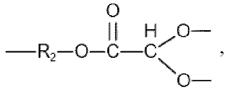 and 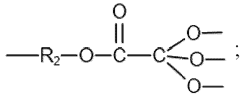 and the imino-containing group comprises any one of - R₃-CH₂-NH-, 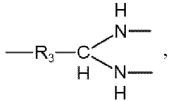 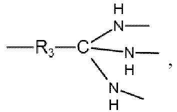 and -R₃-C(=O)-NH-, wherein R₁, R₂, and R₃ are a substituted or unsubstituted alkylene group.

5. The nanocrystalline complex according to claim 3 or 4, wherein a number of carbon atoms in the linking group Y is 1 to 30.

6. The nanocrystalline complex according to claim 1, wherein the linking group Y' comprises one or more of an oxygen atom, a sulfur atom, a substituted or unsubstituted alkylene group, a substituted or unsubstituted alkyleneoxy group, an ester-containing group, and an amido bond-containing group.

7. The nanocrystalline complex according to claim 6, wherein a number of carbon atoms in the linking group Y' is 0 to 30.

8. The nanocrystalline complex according to any one of claims 1 to 7, wherein in R, R', and R", the substituted or unsubstituted alkyl group is a substituted or unsubstituted C1-C20 alkyl group, the substituted or unsubstituted alkoxy group is a substituted or unsubstituted C1-C20 alkoxy group, the substituted or unsubstituted alkyl carbonyl group is a substituted or unsubstituted C1-C20 alkyl carbonyl group, the substituted or unsubstituted cycloalkyl group is a substituted or unsubstituted C3-C20 cycloalkyl group, the substituted or unsubstituted aryl group is a substituted or unsubstituted C6-C20 aryl group, and the substituted or unsubstituted aryloxy group is a substituted or unsubstituted C6-C20 aryloxy group.

9. The nanocrystalline complex according to any one of claims 1 to 8, wherein the photosensitive ligand with the structure shown in formula (I) comprises any one of compounds shown in formula (I-1) to formula (I-14): 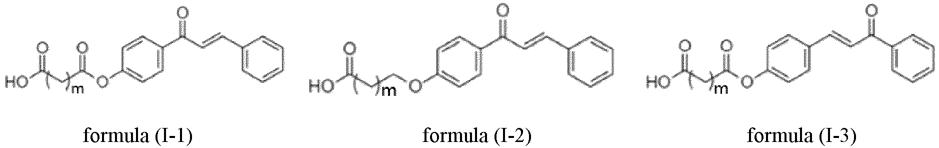 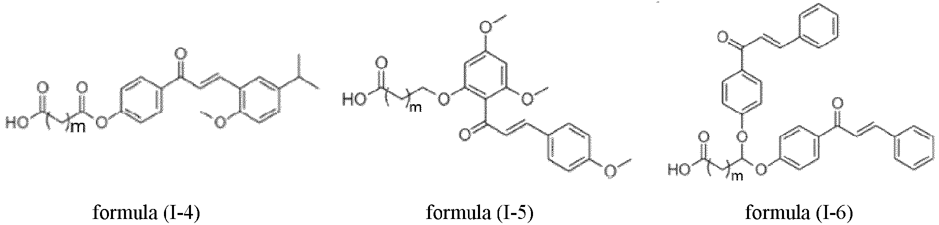 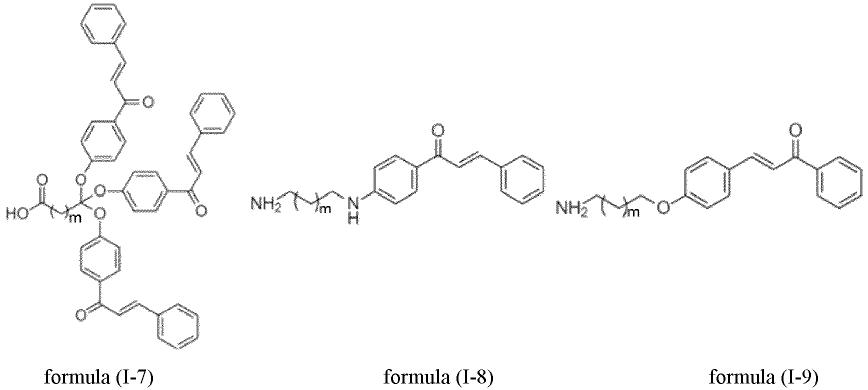 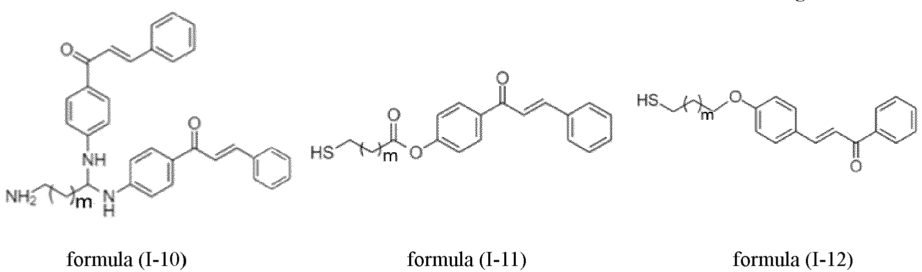 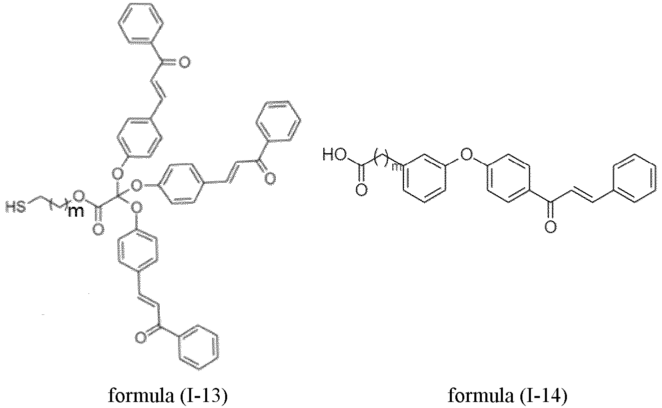 in formula (I-1) to formula (I-14), m is an integer greater than or equal to 0.

10. The nanocrystalline complex according to any one of claims 1 to 8, wherein the photosensitive ligand with the structure shown in formula (II) comprises any one of compounds shown in formula (II-1) to formula (II-12): 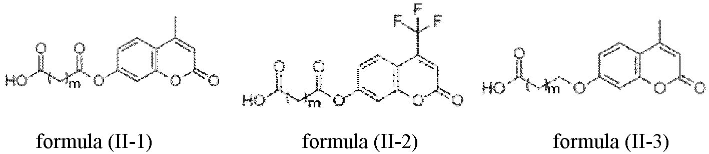 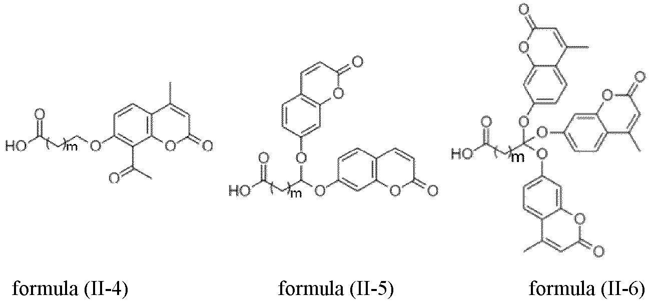 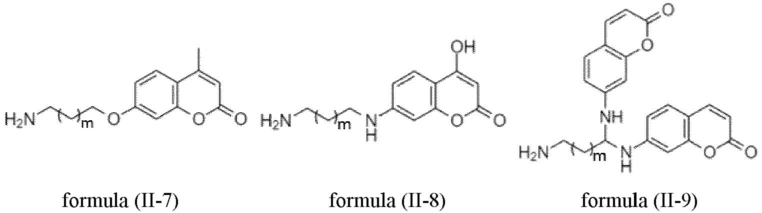 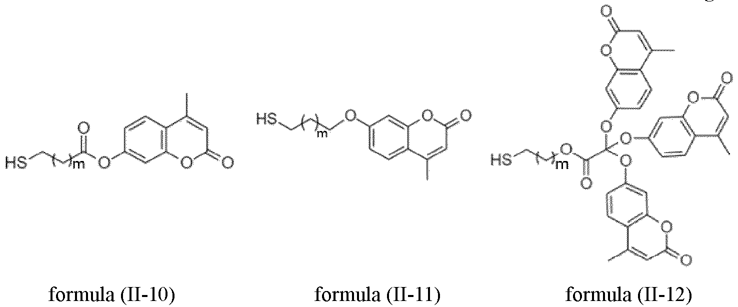 in formula (II-1) to formula (II-12), m is an integer greater than or equal to 0.

11. The nanocrystalline complex according to any one of claims 1 to 10, wherein the nanocrystalline particles comprise one or more of a group II-VI compound, a group IV-VI compound, a group III-V compound, a group III-VI compound, a group VIB-VIA compound, a group VIII-VI compound, a group I-VI compound, a group I elementary substance, a group IV elementary substance, a group I-IV-VII compound, and a perovskite compound.

12. The nanocrystalline complex according to any one of claims 1 to 11, wherein a particle size of the nanocrystalline particles is 5 nm to 20 nm.

13. The nanocrystalline complex according to any one of claims 1 to 12, wherein a total mass of the ligand on the surface of the nanocrystalline particles is 1% to 30% of a mass of the nanocrystalline particles.

14. A method for preparing the nanocrystalline complex according to any one of claims 1 to 13, comprising:
dispersing nanocrystalline particles whose surface is coordinated with a first ligand in a solvent, adding the photosensitive ligand with the structure shown in formula (I) and/or formula (II), and stirring to undergo a reaction, to obtain ligand-exchanged nanocrystalline particles, that is, to obtain the nanocrystalline complex.

15. The preparation method according to claim 14, wherein the first ligand comprises a saturated or unsaturated aliphatic carboxylic acid ligand.

16. The preparation method according to claim 14 or 15, wherein the solvent comprises one or more of chloroform, chlorobenzene, and ethyl benzoate.

17. A nanocrystalline composition, wherein the nanocrystalline composition comprises the nanocrystalline complex according to any one of claims 1 to 13 and a solvent.

18. The nanocrystalline composition according to claim 17, wherein in the nanocrystalline composition, a mass concentration of the nanocrystalline complex is 1 mg/mL to 100 mg/mL.

19. The nanocrystalline composition according to claim 17 or 18, wherein the solvent comprises one or more of chloroform, chlorobenzene, and ethyl benzoate.

20. A method for preparing a patterned film, comprising:
providing a solution containing the nanocrystalline complex according to any one of claims 1 to 13 or providing the nanocrystalline composition according to any one of claims 17 to 19 on a substrate, and performing exposure and development by using a mask, wherein the nanocrystalline complex in an exposure region undergoes photo-crosslinking to be attached to the substrate, and the nanocrystalline complex in a non-exposure region is removed through the development, to form the patterned film.

21. The preparation method according to claim 20, wherein the exposure is performed through radiation of ultraviolet light with a light intensity of 0.001 mW/cm² to 1000 mW/cm².

22. The preparation method according to claim 20 or 21, wherein time for the exposure is 0.1 seconds to 600 seconds.

23. The preparation method according to any one of claims 20 to 22, wherein the development is development with a solvent, and an organic solvent capable of eluting the nanocrystalline complex is used for the development.

24. A patterned film formed through crosslinking of the nanocrystalline complex according to any one of claims 1 to 13; or a patterned film prepared by the preparation method according to any one of claims 20 to 23.

25. An electronic component, wherein the electronic component comprises a first electrode and a second electrode that are opposite to each other, and a functional layer between the first electrode and the second electrode, wherein the functional layer comprises a crosslinking product of the nanocrystalline complex according to any one of claims 1 to 13; or the functional layer comprises the patterned film according to claim 24.

26. The electronic component according to claim 25, wherein the electronic component comprises any one of an LED, a QLED, a mini-LED, a micro-LED, a nano-LED, and a QD-OLED.

27. A display apparatus, wherein the display apparatus comprises the electronic component according to claim 25 or 26.

28. An electronic device, wherein the electronic device comprises the display apparatus according to claim 27.
